# EUROPEAN PATENT SPECIFICATION

(11) **EP 1 873 160 B1**
(45) Date of publication and mention of the grant of the patent: **13.07.2011**
(21) Application number: 06729507.1
(22) Date of filing: 20.03.2006
(51) Int. Cl.: C07F 9/40, C07F 5/06, C07C 251/24, C07C 249/02, B01J 31/22, C07B 53/00, C07B 61/00

(54) **PROCESS FOR PRODUCTION OF OPTICALLY ACTIVE -HYDROXYPHOSPHONIC ACIDS AND DERIVATIVES THEREOF, OPTICALLY ACTIVE ALUMINUM(SALALEN) COMPLEXES AND PROCESS FOR PRODUCTION OF THE COMPLEXES, AND PROCESS FOR PRODUCTION OF SALALEN LIGANDS**
VERFAHREN ZUR HERSTELLUNG VON OPTISCH AKTIVEN HYDROXYPHOSPHONSÄUREN UND DERIVATEN DAVON, OPTISCH AKTIVE ALUMINIUM (SALALEN)-KOMPLEXE UND VERFAHREN ZUR HERSTELLUNG DER KOMPLEXE, UND VERFAHREN ZUR HERSTELLUNG VON SALALENLIGANDEN
PROCEDE DE PRODUCTION D ACIDES -HYDROXYPHOSPHONIQUES OPTIQUEMENT ACTIFS ET LEURS DERIVES, COMPLEXES D ALUMINIUM(SALALEN) OPTIQUEMENT ACTIFS ET PROCEDE DE PRODUCTION DES COMPLEXES, ET PROCEDE DE PRODUCTION DES LIGANDS DE SALALEN

(30) Priority: 12.04.2005 JP 2005114484
(43) Date of publication of application: 02.01.2008
(73) Proprietor: Japan Science and Technology Agency, Kawaguchi-shi, Saitama 332-0012 (JP)
(72) Inventor: KATSUKI, Tsutomu, Fukuoka-shi, Fukuoka 813-0012 (JP); SAITO, Bunnai, Fukuoka-shi, Fukuoka 819-0166 (JP)
(74) Representative: Westwood, Joanna
(86) International application number: PCT/JP2006/305539
(87) International publication number: WO 2006/109442

(56) References cited:
- JP-A- 08 319 258
- JP-A- 2002 513 734
- JP-A- 2003 183 252
- JP-B1- 46 021 933
- KEE T P ET AL: "The Asymmetric Phospho-Aldol Reaction. Past, Present and Future" TOPICS IN CURRENT CHEMISTRY, SPRINGER, BERLIN, DE, vol. 223, 1 January 2003 (2003-01-01), pages 45-65, XP008096600 ISSN: 0340-1022
- WARD, CAROLINE V. ET AL: "New chiral catalysts for phospho-transfer" TETRAHEDRON LETTERS , 41(32), 6181-6184 CODEN: TELEAY; ISSN: 0040-4039, 2000, XP002496144
- YEORI A. ET AL.: 'Salalen: a hybrid Salan/Salen tetradentate [ONNO]-type ligand and its coordination behavior with group IV metals' INORG. CHEM. COMMUN. vol. 7, 2004, pages 280 - 282, XP003000296
- SAITO B. ET AL.: 'Synthesis of an Optically Active C1-Symmetric Al(salalen) Complex and Its Application to the Catalytic Hydrophosphorylation of Aldehydes' ANGEW. CHEM. INT. ED. vol. 44, 18 July 2005, pages 4600 - 4602, XP003000297

## Description

### FIELD OF THE INVENTION

The present invention relates to the production method of optically active α-hydroxyphosphonic acid and its derivatives, optically active aluminum(salalen) complex suitable as the catalyst for the said production method and the production method thereof, as well as the production method of the salalen ligand that may be used for the production of the said complex, and more specifically, a method for the production of optically active α-hydroxyphosphonic acid or its derivatives through asymmetric hydrophosphonylation of aldehydes by phosphonic acid or its derivative using optically active aluminum(salalen) complexes having a specific structure as the catalyst.

### BACKGROUND OF THE INVENTION

In the past several decades, asymmetric catalysis by optically active complexes have been a major subject in the field of synthetic chemistry, and various chiral ligands comprising said optically active complexes have been developed. Out of such ligands, quadridentate ligands are widely used because of their high complex formation capability. Especially, out of such quadridentate ligands, the salen ligand has received broad attention because of its high-level asymmetric induction capability as well as ease-of acquisition. For example, chiral metallosalen complexes, because of its high asymmetric catalysis activity and because it shows catalysis activity for various asymmetric reactions, are being used as the catalyst for various asymmetric reactions such as epoxydation, aziridination, sulfoxidation, Michael reaction, epoxy ring-opening reaction, and the like. Here, most salen complexes have a octahedral structure having two trans-oriented ancillary ligands. However, recent research has revealed that the cis-β-isomer shows a unique catalytic ability. For example, the di-µ-oxo Ti (salen) complex having chiral cis-β salen ligands shows high enantioselectivity for asymmetric cyanization. Also the di-µ-oxo Ti (salen) complex is known to work as the active specie for asymmetric sulfoxidation. Also, Kol and coworkers have, very recently, reported that by treating achiral hybrid salan/salen [ONN(Me)O]- type quadrdentate ligand (in the following, this will be referred to as salalen ligand) with titanium tetraethoxide and zirconium tetraethoxide, corresponding octahedral structured Ti (salalen) (OEt)₂ and Zr (salalen) (OEt)₂ complexes are each formed, that in the said complexes, diastereotopic ethoxy groups are cis-oriented, and that unlike previously known salen complexes, the coordinating amino nitrogen atom that is not just near the ethylene carbon but closer to the metal ion is chiral (see A. Yeori, S. Gendler, S. Groysman, I. Goldberg, M. Kol, Inorg. Chem. Commun., 2004, 7, 280-282).

Now, optically active α-hydroxyphosphonic ester and phosphonic acid are bioactive compounds used widely for medication uses, and in order to produce said optically active α-hydroxyphosphonic ester and phosphonic acid efficiently, there has been a lot of energy put into the development of asymmetric hydrophosphonylation of carbonyl compounds (Pudovik reaction). Moreover, today, the most effective catalysts for the said asymmetric hydrophosphonylation are lanthanum tris(binaphthoxide) complex and aluminum tris(binaphtoxide) complex developed by Shibasaki and coworkers (see T. Arai, M. Bougauchi, H. Sasai, M. Shibasaki, J. Org. Chem., 1996, 61, 2926-2927; and H. Sasai, M. Bougauchi, T. Arai, M. Shibasaki, Tetrahedron Lett., 1997, 38, 2717-2720). However, although lanthanum tris(binaphthoxide) complex and aluminum tris(binaphothoxide) complex show enough enantioselectivity for aromatic aldehydes, there is a problem that enantioselectivity is low for aliphatic aldehydes. Also, recently, Kee and coworkers have reported that chiral Al(salen) complexes show catalysis for asymmetric hydrophosphonylation, but the enantioselectivity is around 49%ee and low (see J. P. Duxbury, A. Cawley, M. Thornton-Pett, L. Wantz, J. N. D. Warne, R. Greatrex, D. Brown, T. P. Kee, Tetrahedron Lett., 1999, 40, 4403-4406; C.V. Ward, M. Jiang, T.P. Kee, Tetrahedron Lett., 2000, 41, 6181-6184; and J. P. Duxbury, J. N. D. Warne, R. Mushtaq, C. Ward, M. Thornton-Pett, M. Jiang, R. Greatrex, T. P. Kee, Organometallics, 2000, 19,4445-4457), and more improvement is desired. Further examples of asymetric phospho-aldol reactions also exist (T. P Kee, T. D. Nixon, Topics in Current Chemistry, 2003,223,45-65).

### DISCLOSURE OF THE INVENTION

Under these circumstances, the object of the present invention is to solve the problems mentioned above and to provide a production method that allows the synthesis of optically active α-hydroxyphosphonic acid and its derivatives with sufficiently high enantioselectivity not only for aromatic aldehydes but also for aliphatic aldehydes. Also, another object of the present invention is to provide new complexes effective as catalysts for the said production method as well as their production method.

The inventers of the present invention have found, as a result of keen examination for accomplishing the above object, that by using optically active aluminum(salalen) complexes having a specific structure as the catalyst to asymmetrically hydrophosphonylate aldehydes by phosphonic acid or its derivatives, optically active α-hydroxyphosphonic acid or its derivatives can be produced with high enantioselectivity and completed the present invention.

That is, the production method of the optically active α-hydroxyphosphonic acid or its derivatives according to the present invention is characterized in that an optically active aluminum(salalen) complex represented by any of the following formulae (I), (I'), (II) and (II'): 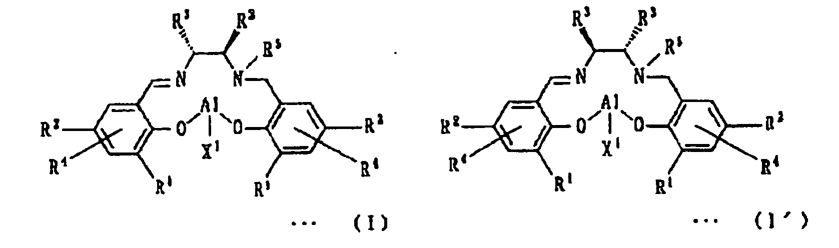 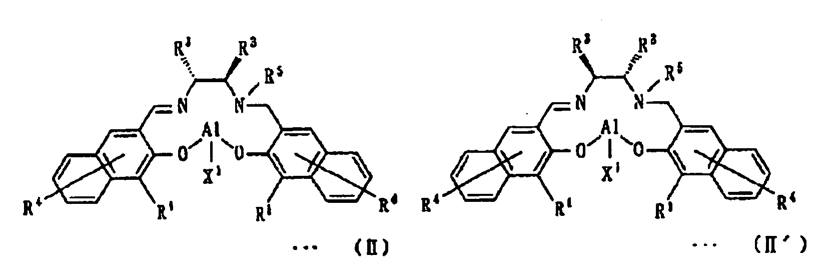 [wherein R¹s are each alkyl group or aryl group independently; R²s are each alkyl group or aryl group independently; R³s are each alkyl group or aryl group independently, and two R³s may bond with each other to form a ring; R⁴s are each hydrogen atom, halogen atom, alkyl group, alkoxy group, nitro group, or cyano group independently; R⁵ is alkyl group; and X¹ is halogen atom, alkyl group, alkoxy group, acetoxy group or toluenesulfonyloxy group] is used as a catalyst and an aldehyde represented by the following formula (III): 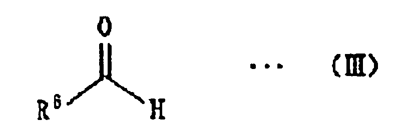 [wherein R⁶ is a monovalent group] is asymmetrically hydrophosphonylated with a phosphonic acid or its derivative represented by the following formula (IV): 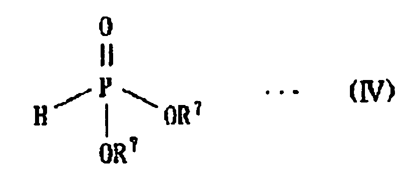 [wherein R⁷s are each hydrogen atom or monovalent group independently] to produce an optically active α-hydroxyphosphonic acid or its derivatives represented by the following formula (V): 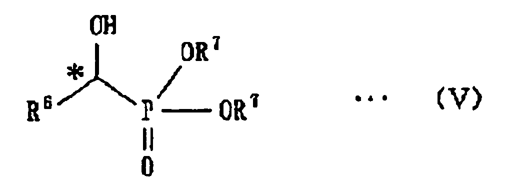 [wherein R⁶ and R⁷s are the same as above].

In a preferable embodiment of the production method of the optically active α-hydroxyphosphonic acid or its derivatives in the present invention, the two R³s in the above formulae are bonded with each other to form a tetramethylene group. Also, as the optically active aluminum(salalen) complex used as the catalyst, the complexes represented by the above formula (I) or (I') are preferable, and it is more preferable that R¹s and R²s in the said formula are t-butyl groups. Also, it is preferable that R⁵ in the above formulas is methyl group.

In another preferable embodiment of the production method of the optically active α-hydroxyphosphonic acid or its derivatives in the present invention, R⁶ in the above formula (III) is a monovalent hydrocarbon group. In this case, the enantiomer excess of the product can be improved.

In the other preferable embodiment of the production method of the optically active α-hydroxyphosphonic acid or its derivatives in the present invention, R⁷s in the above formula (IV) are alkyl group or aryl group. In this case, the enantiomer excess of the product can be improved.

Also, the optically active aluminum(salalen) complexes of the present invention is characterized in that they are represented by any one of the above formulae (I), (I'), (II) and (II').

In a preferable embodiment of the optically active aluminum(salalen) complexes of the present invention, the two R³s of the above formulae are bonded with each other to form a tetramethylene group. Also, as the optically active aluminum(salalen) complexes of the present invention, complexes represented by the above formula (I) or (I') are preferable, and it is more preferable that R¹s and R²s in the said formula are t-butyl groups. Also, it is preferable that R⁵ in the above formula is methyl group.

Moreover, the production method of the optically active aluminum(salalen) complexes of the present invention is characterized in that a salalen ligand represented by any one of the following formulae (VI), (VI'), (VII) and (VII'): 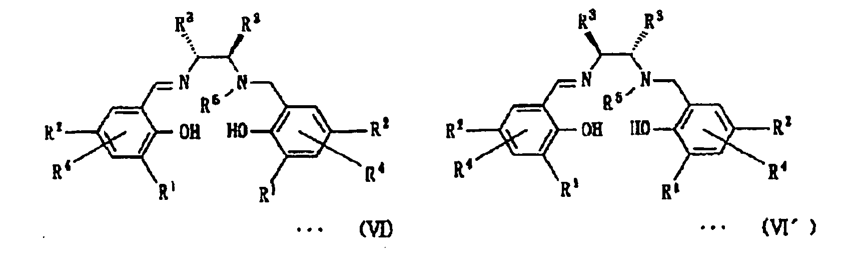 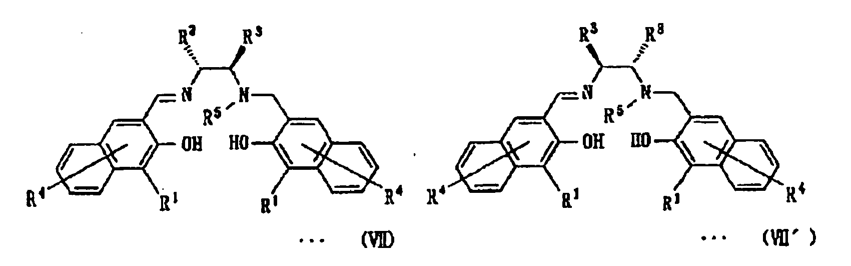 [wherein R¹, R², R³, R⁴, and R5 are the same as above] is reacted with an aluminum compound represented by the following formula (VIII-a) or (VIII-b):

R⁸₂ A I X¹ (VIII-a)

R⁸₃ A I (VIII-b)

[wherein R⁸s are each alkyl group independently; and X¹ is the same as above] to produce an optically active aluminum(salalen) complex represented by any one of the above formulae (I), (I'), (II) and (II').

In a preferable embodiment of the production method of the optically active aluminum(salalen) complex of the present invention, the two R³s in the above formulae are bonded with each other to form a tetramethylen group. Also, it is preferable that the above salalen ligand is represented by above formula (VI) or (VI'), and that the above optically active aluminum(salalen) complex is represented by the above formula (I) or (I'), and it is more preferable that R¹s and R²s in the said formula are t-butyl groups. Also, it is preferable that R⁵ in the above formula is methyl group.

Furthermore, the salalen ligands of the present invention are characterized in that they are represented by any one of the above formulae (VI), (VI'), (VII) and (VII').

In a preferable embodiment of the salalen ligands of the present invention, the two R³s in the above formulae are bonded with each other to form a tetramethylene group. Also, as the salalen ligands of the present invention, salalen ligands represented by above formulas (VI) or (VI') is preferable, and it is more preferable that R¹s and R²s in the said formula are t-butyl groups. Also, it is more preferable that the R⁵ in the above formulas is methyl group.

Furthermore, the production method of the salalen ligands of the present invention is characterized by comprising (i) a step of reductively aminating an aldehyde represented by the following formula (IX) or (X): 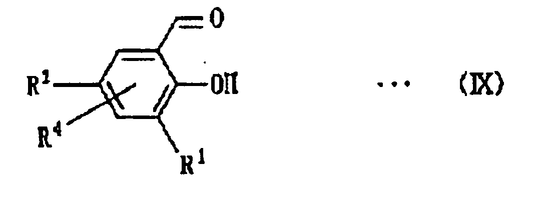 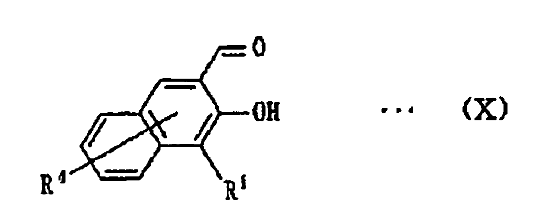 [wherein R¹, R², and R⁴ are the same as above] with a monoammonium salt of a diamine represented by the following formulas (XI) or (XI'): 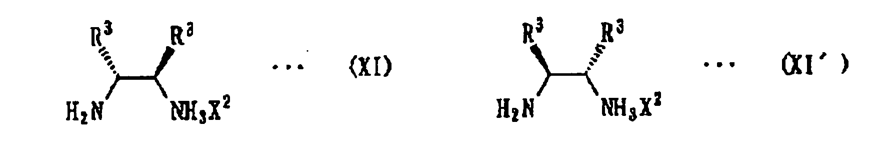 [wherein R³s are the same as above, X² is halogen atom, alkyl group, alkoxy group, acetoxy group, or toluenesulfonyloxy group] and a reducing agent to form a compound represented by any one of the following formulae (XII), (XII'), (XIII) and (XIII'): 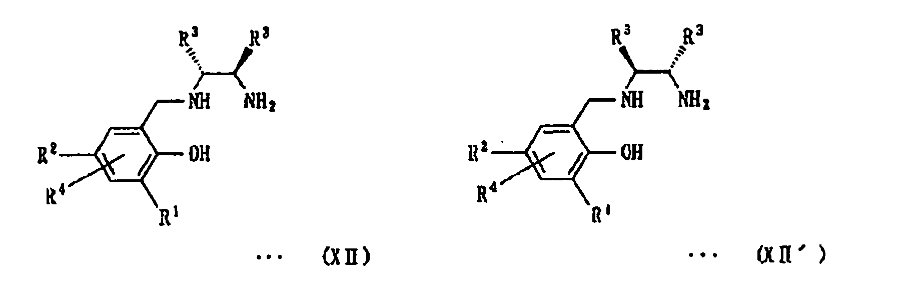 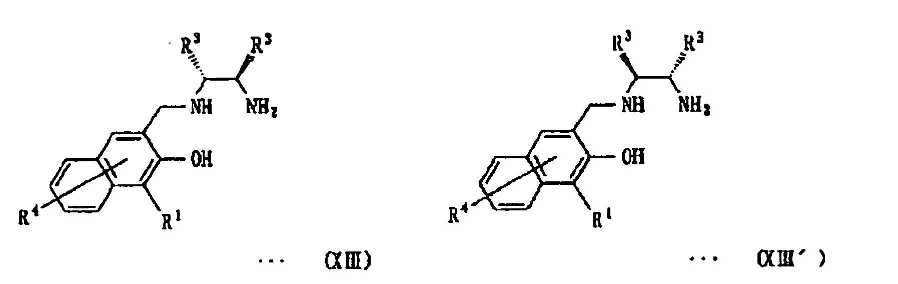 [wherein R¹, R², R³, and R⁴ are the same as above];
(ii) a step of protecting an amino group of the compound represented by any one of the above formulae (XII), (XII'), (XIII) and (XIII') with a protecting group to form a compound represented by any one of the following formulae (XIV), (XIV'), (XV) and (XV'): 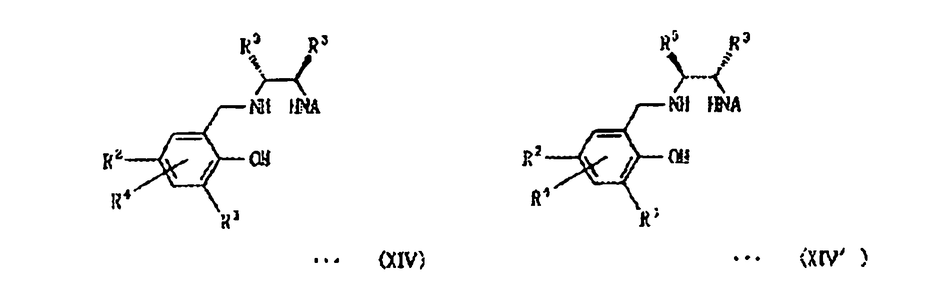 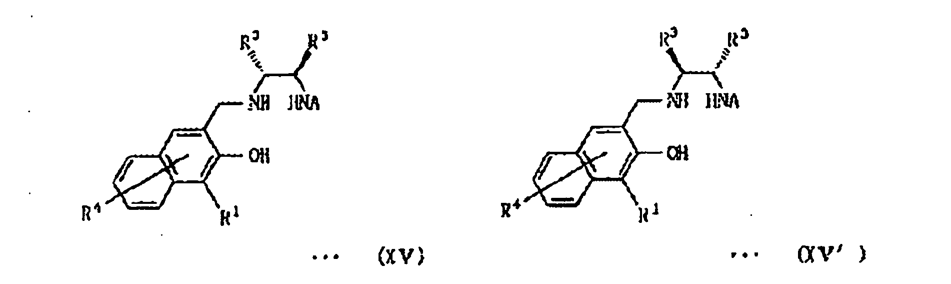 [wherein R¹, R², R³, and R⁴ are the same as above and A is the protecting group];
(iii) a step of N-alkylating the compound represented by any one of the above formulae (XIV), (XIV'), (XV) and (XV') to form a compound represented by any one of the following formulae (XVI), (XVI'), (XVII) and (XVII'): 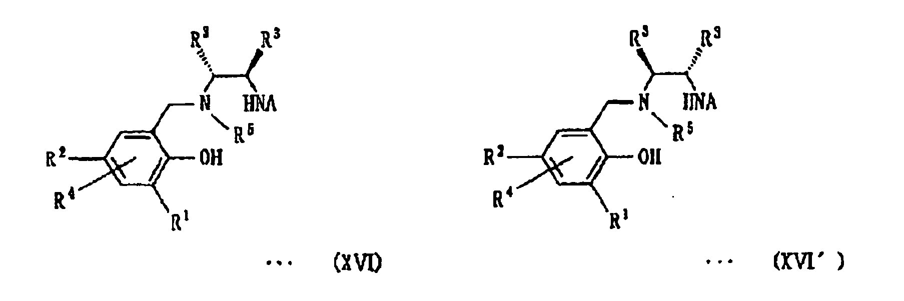 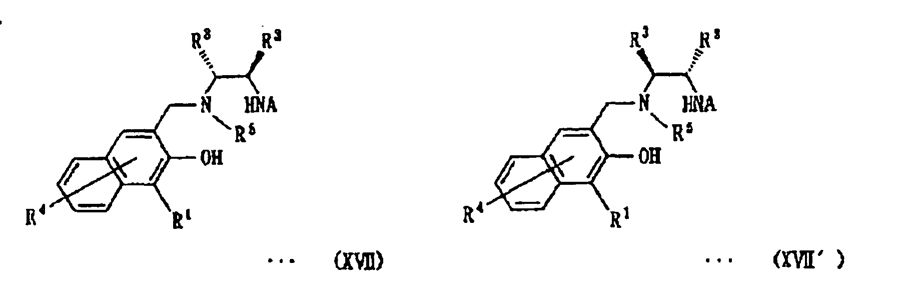 [wherein R¹, R², R³, R⁴ and A are the same as above];
(iv) a step of deprotecting the protecting group in the compound represented by any one of the above formulae (XVI), (XVI'), (XVII) and (XVII') to form a compound represented by any one of the following formulae (XVIII), (XVIII'), (XIX) and or (XIX'): 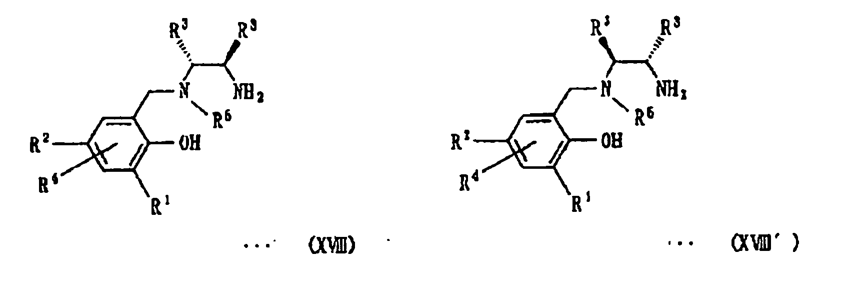 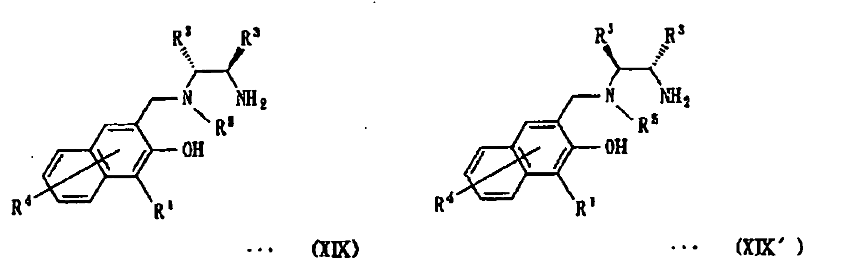 [wherein R¹, R², R³, R⁴ and R⁵, are the same as above], and
(v) a step of condensing the compound represented by any one of the above formulae (XVIII), (XVIII'), (XIX) and (XIX') with the aldehyde represented by the above formulas (IX) or (X) to form a salalen ligand represented by any one of the above formulae (VI), (VI'), (VII) and (VII').

In a preferable embodiment of the production method of the salalen ligands of the present invention, the two R³s in the above formulae are bonded with each other to form a tetramethylene group. Also, as the salalen ligand to be formed, the salalen ligand represented by the above formula (VI) or (VI') is preferable, and it is more preferable that R¹s and R²s in the said formula are t-butyl groups. Also, it is preferable that the N-alkylation in the above step (iii) is N-methylation and that R⁵ in the above formula is methyl group.

According to the present invention, it is possible to produce optically active α-hydroxyphosphonic acid or its derivatives with high enantioselectivity by using optically active aluminum(salalen) complexes having specific structures as the catalyst to asymmetrically hydrophosphonylate aldehydes by phosphonic acid or its derivatives.

### BRIEF EXPLANATION OF THE DRAWINGS

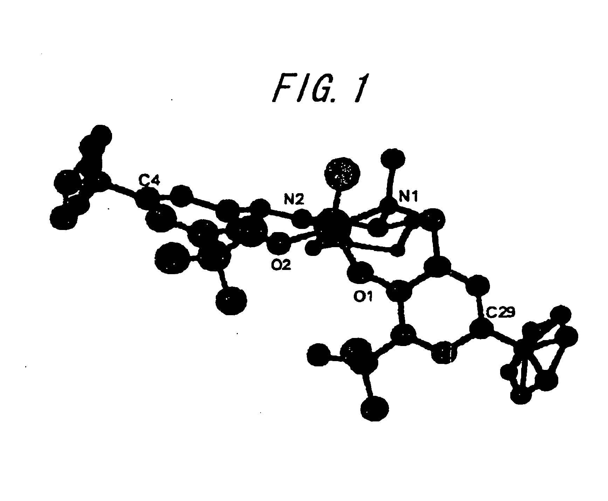

FIG 1 shows results of an X-ray structure analysis of the crystal obtained by recrystallizing the aluminum(salalen) complex represented by formula (XXI) from heptane/dichloromethane.

### BEST MODE FOR CARRYING OUT THE INVENTION

Below, the present invention will be explained in detail. The optically active aluminum(salalen) complex is represented by any one of the above formulas (I), (I'), (II) and (II'). Here, the complex of formula (I') is the enantiomer of the complex of formula (I), and the complex of formula (II') is the enantiomer of the complex of formula (II) and can be synthesized in the same manner by choosing the configuration of the starting material. Out of these, the complexes represented by formulas (I) and (I') are preferable. The amount of the above complexes used is, in relation to the molar quantity of the aldehyde of the substrate to be explained below, preferably in the range of 0.01 - 100 mol%, and more preferably in the range of 0.1 - 10 mol%.

The above aluminum(salalen) complexes are, according to the result of X-ray structure analysis (the result of the X-ray structure analysis of an example of the aluminum(salalen) complexes of the present invention is shown in Fig. 1), configured in a distorted trigonal bipyramid structure, and have a structure that is different from aluminum(salen) complexes heretofore known. The N-alkyl group in the said complexes (that is, R⁵) is cis-oriented to X¹. Moreover, said X¹ is, when the above aluminum(salalen) complexes act as Lewis acid catalyst, substituted by the substrate. Therefore, the above aluminum(salalen) complexes can be regarded to be good as a chiral Lewis acid catalyst. Furthermore, by using the said aluminum(salalen) complexes as the catalyst for asymmetric hydrophosphonylation of aldehydes, α-hydroxyphosphonic acid or its derivatives can be produced with high enantioselectivity.

The R¹s in the above formulae are each alkyl group or aryl group independently, and as the said alkyl group are given alkyl groups having 1-6 carbon atoms such as methyl group, ethyl group, n-propyl group, i-propyl group, n-butyl group, i-butyl group, s-butyl group, t-butyl group, 1,1-dimethylpropyl group, 1-ethyl-1-methyl-propyl group and the like, and as the aryl group are given aryl groups having 6-22 carbon atoms such as phenyl group, 3,5-dimethylphenyl group, 4-methylphenyl group, 1-naphthyl group, 2-biphenyl group, 2-phenyl-1-naphthyl group, 2-methyl-1-naphthyl group, 2-[3,5-dimethylphenyl]-1-naphthyl group, 2-[4-methylphenyl]-1-naphthyl group, 2-methoxy-1-naphthyl group, 2-[p-(t-butyldimethylsilyl)phenyl]-1-naphthyl group, 2-biphenyl-1-naphthyl group and the like. Moreover, the above aryl group may be optically active, or optically inactive. Here, as R¹, t-butyl group is preferable.

Also, the R²s in the above formulae are each alkyl group or aryl group independently, and as the said alkyl group are given alkyl groups having 1- 6 carbon atoms such as methyl group, ethyl group, n-propyl group, i-propyl group, n-butyl group, i-butyl group, s-butyl group, t-butyl group, 1,1-dimethylpropyl group, 1-ethyl-1-methylpropyl group and the like, and as the aryl group are given aryl groups having 6-18 carbon atoms such as phenyl group, 3,5-dimethylphenyl group, 4-methylphenyl group, 1-naphthyl group, 2-biphenyl group, 2-phenyl-1-naphthyl group, 2-methyl-1-naphthyl group, 2-[3,5-dimethylphenyl]-1-naphthyl group, 2-[4-methylphenyl]-1-naphthyl group, 2-methoxy-1-naphthyl group and the like. Here, as R², t-butyl group is preferable.

Furthermore, the R³s in the above formulas are each alkyl group of aryl group independently, and the two R³s may be bonded with each other to form a ring. As the said alkyl group are given alkyl groups having 1- 4 carbon atoms such as methyl group, ethyl group, n-propyl group, i-propyl group, n-butyl group, i-butyl group, s-butyl group, t-butyl group and the like, and as the aryl group are given aryl groups having 6-18 carbon atoms such as phenyl group, 3,5-dimethylphenyl group, 4-methylphenyl group, 1-naphthyl group, 2-biphenyl group, 2-phenyl-1-naphthyl group, 2-methyl-1-naphthyl group, 2-[3,5-dimethylphenyl]-1-naphthyl group, 2-[4-methylphenyl]-1-naphthyl group, 2-methoxy-1-naphthyl group and the like. Also, when the two R³s are bonded with each other to form a ring, as the bivalent group that is formed is given tetramethylene group and the like. Out of these, it is preferable that the two R³s are bonded with each other to form tetramethylene group.

Moreover, the R⁴s in the above formulae are each hydrogen atom, halogen atom, alkyl group, alkoxy group, nitro group, or cyano group independently. Here, as the halogen atom are given fluorine atom, chlorine atom, bromine atom and the like, and as the alkyl group are preferable alkyl groups having 1-4 carbon atoms such as methyl group, ethyl group, n-propyl group, i-propyl group, n-butyl group, i-butyl group, s-butyl group, t-butyl group and the like, and as the alkoxy group are preferable alkoxy groups having 1-4 carbon atoms such as methoxy group, ethoxy group, n-propoxy group, i-propoxy group, n-butoxy group, i-butoxy group, s-butoxy group, t-butoxy group and the like. Out of these, as R⁴, hydrogen atom is especially preferable.

Moreover, the R⁵ in the above formulae is alkyl group, and as the said alkyl group are given alkyl groups having 1- 4 carbon atoms such as methyl group, ethyl group, n-propyl group, i-propyl group, n-butyl group, i-butyl group, s-butyl group, t-butyl group and the like, and out of these, methyl group is preferable.

Also, X¹ in the above formulas is halogen atom, alkyl group, alkoxy group, acetoxy group, or toluenesulfonyloxy group, and as the above halogen atom are given fluorine atom, chlorine atom, bromine atom and the like, as the above alkyl group are given methyl group, ethyl group, n-propyl group, i-propyl group, n-butyl group, i-butyl group, s-butyl group, t-butyl group and the like, and as the above alkoxy group are given methoxy group, ethoxy group, n-propoxy group, i-propoxy group, n-butoxy group, i-butoxy group, s-butoxy group, t-butoxy group and the like. Out of these, as X¹, chlorine atom is preferable.

The optically active aluminum(salalen) complex represented by any one of the above formulae (I), (I'), (II) and (II') can, for example, be produced by reacting a salalen ligand represented by any one of the above formulae (VI), (VI'), (VII) and (VII') with an aluminum compound represented by the above formula (VIII-a) or (VIII-b). Here, R¹s, R²s, R³s, R⁴s, and R⁵ in formulae (VI), (VI'), (VII) and (VII') as well as X¹ in formulae (VIII-a) and (VIII-b) are the same as above, and R⁸s in formulae (VIII-a) and (VIII-b) are each alkyl group independently. As the alkyl group of R⁸s in formulae (VIII-a) and (VIII-b) are preferable alkyl groups having 1- 4 carbon atoms such as methyl group, ethyl group, n-propyl group, i-propyl group, n-butyl group, i-butyl group, s-butyl group, t-butyl group and the like, and out of these, ethyl group is especially preferable. Also, the amount of the aluminum compound of formula (VIII-a) or (VIII-b) used is, relative to the molar quantity of the above salalen ligand, preferably in the range of 100 - 200 mol%. Furthermore, the above reaction is preferably performed, for example, in an organic solvent such as toluene and the like, at 0°C - room temperature.

In the production method of the optically active aluminum(salalen) complexes of the present invention, the complex of formula (I) can be produced by using the salalen ligand of formula (VI), and the complex of formula (I') can be produced by using the salalen ligand of formula (VI'), and the complex of formula (II) can be produced by using the salalen ligand of formula (VII), and the complex of formula (II') can be produced by using the salalen ligand of formula (VII').

The salalen ligands represented by any one of the above formulae (VI), (VI'), (VII) and (VII') can, for example, be produced through the following five steps.

First, in step (i), an aldehyde represented by the above formula (IX) or (X) is reductively aminated with a monoammonium salt of a diamine represented by the above formula (XI) or (XI') and a reducing agent to form a compound represented by any one of the above formulae (XII), (XII'), (XIII) and (XIII'). Here, R¹, R², and R⁴ in the formulae (IX) and (X), R³s in the formulae (XI) and (XI'), and R¹, R², R³s and R⁴ in the formulae (XII), (XII'), (XIII) and (XIII') are the same as above, and X² in the formulae (XI) and (XI') is halogen atom, alkyl group, alkoxy group, acetoxy group, or toluenesulfonyloxy group, and as the above halogen atom are given fluorine atom, chlorine atom, bromine atom and the like, and as the above alkyl group are given methyl group, ethyl group, n-propyl group, i-propyl group, n-butyl group, i-butyl group, s-butyl group, t-butyl group and the like, and as the above alkoxy group are given methoxy group, ethoxy group, n-propoxy group, i-propoxy group, n-butoxy group, i-butoxy group, s-butoxy group, t-butoxy group and the like. Out of these, as X², chlorine atom is preferable. The above step (i) is preferably performed, for example, with existence of a reducing agent such as NaBH₄ in a solvent such as methanol at 0°C - room temperature. Also, the amount of monoammonium salt of a diamine represented by the formula (XI) or (XI') and the reducing agent used is, in relation to the molar quantity of the aldehyde of the above formula (IX) or (X), preferably in the range of 100 -200 mol%.

Next, in step (ii), an amino group of the compound represented by any one of the above formulae (XII), (XII'), (XIII) and (XIII') is protected with a protecting group to form a compound represented by any one of the above formulae (XIV), (XIV'), (XV) and (XV'). Here, R¹, R², R³s, and R⁴ in formulae (XIV), (XIV'), (XV) and (XV') are the same as above, and A is the protecting group. As the said protecting group are given t-butoxycarbonyl (Boc) group, benzyloxycarbonyl group, t-amyloxycarbonyl group and the like, and out of these, t-butoxycarbonyl (Boc) group is preferable. Moreover, the protecting reagent used to introduce the protecting group is not limited specifically, and protecting reagents heretofore known can be used. The above step (ii) is preferably performed, for example, in a solvent such as ethanol at room temperature. Furthermore, the amount of protecting reagent used is, in relation to the molar quantity of the compound of the above formulas (XII), (XII'), (XIII), or (XIII'), preferably in the range of 100-200 mol%.

Next, in step (iii), the compound represented by any one of the above formulae (XIV), (XIV'), (XV) and (XV') is N-alkylated to form a compound represented by any one of the above formulae (XVI), (XVI'), (XVII) and (XVII'). Here, R¹, R², R³s, R⁴, R⁵, and A in the formulae (XVI), (XVI'), (XVII) and (XVII') are the same as above. The above step (iii) is preferably performed, for example, in the case of N-methylation, using formaldehyde aqueous solution, Pd/C, and H₂ in a solvent such as methanol at room temperature. Also, the amount of the reagents used for N-alkylation of formaldehyde and the like is, in relation to the molar quantity of the compound of the above formula (XIV), (XIV'), (XV) or (XV'), preferably in the range of 100 - 200 mol%.

Next, in step (iv), the protecting group in the compound represented by any one of the above formulae (XVI), (XVI'), (XVII) and (XVII') is deprotected to form a compound represented by any one of the above formulae (XVIII), (XVIII'), (XIX) and (XIX'). Here, R¹, R², R³s, R⁴, and R⁵ in the formulae (XVIII), (XVIII'), (XIV) and (XIV') are the same as above. The above step (iv) is preferably performed, for example, in an acid such as hydrochloric acid at room temperature.

Lastly, in step (v), the compound represented by any one of the above formulae (XVIII), (XVIII'), (XIX) and (XIX') and an aldehyde represented by the above formulas (IX) or (X) are condensed to form a salalen ligand represented by any one of the above formulae (VI), (VI'), (VII) and (VII'). The above step (v) is preferably performed, for example, in a solvent such as methanol at room temperature. Also, the amount of the aldehyde of the formula (IX) or (X) used is, in relation to the molar quantity of the compound of the above formula (XVIII), (XVIII'), (XIX) or (XIX'), preferably in the range of 100 - 200 mol%.

In the production method of the salalen ligand of the present invention, the salalen ligand of formula (VI) can be produced by using the aldehyde of formula (IX) and the monoammonium salt of the diamine of formula (XI) as the starting material, and the salalen ligand of formula (VI') can be produced by using the aldehyde of formula (IX) and the monoammonium salt of the diamine of formula (XI') as the starting material, and the salalen ligand of formula (VII) can be produced by using the aldehyde of formula (X) and the monoammonium salt of the diamine of formula (XI) as the starting material, and the salalen ligand of formula (VII') can be produced by using the aldehyde of formula (X) and the monoammonium salt of the diamine of formula (XI') as the starting material.

In the method of producing the optically active α-hydroxyphosphonic acid or its derivatives of the present invention, the aldehyde represented by the above formula (III) and phosphonic acid or its derivative represented by the above formula (IV) are used as the raw materials to form an optically active α-hydroxyphosphonic acid or its derivative. In this reaction, the carbon of the carbonyl in the aldehyde is phosphonylated, and hydrogen is added to the oxygen of the carbonyl.

The R⁶ in above formula (III) is a monovalent group, and as the said monovalent group are given monovalent hydrocarbon groups such as alkyl group, aryl group, alkenyl group, aralkyl group, arylalkenyl group and the like, and hydrogen atoms in these monovalent hydrocarbon groups may be substituted by a substituting group. Here, as the alkyl group are given methyl group, ethyl group, n-propyl group, i-propyl group, n-butyl group, i-butyl group, s-butyl group, t-butyl group, pentyl group, isopentyl group, neopentyl group, hexyl group, heptyl group, octyl group, 2-ethylhexyl group, nonyl group, decyl group, undecyl group, dodecyl group, tridecyl group, isotridecyl group, myristyl group, palmityl group, stearyl group, icosyl group, docosyl group and the like, as the aryl group are given phenyl group, tolyl group, ethylphenyl group, xylyl group, cumenyl group, mesityl group, naphthyl group, biphenyl group and the like, as the alkenyl group are given vinyl group, allyl group, isopropenyl group and the like, as the aralkyl group are given benzyl group, phenethyl group and the like, and as the arylalkenyl group are given styryl group, cinnamyl group and the like. Also, as the substituting group of the above monovalent hydrocarbon group are given halogen atom, nitro group, alkoxy group and the like, and as the said halogen atom are given fluorine atom, chlorine atom, bromine atom and the like, and as the said alkoxy group are given methoxy group, ethoxy group, n-propoxy group, i-propoxy group, n-butoxy group, i-butoxy group, s-butoxy group, t-butoxy group and the like.

The R⁷s in the above formula (IV) is hydrogen atom or monovalent group, and the two R⁷s may be the same or different. Here, as the monovalent group represented by R⁷ in formula (IV) are given monovalent hydrocarbon groups such as alkyl group, aryl group, alkenyl group, aralkyl group, arylalkenyl group and the like, and hydrogen atoms in these monovalent hydrocarbon groups may be substituted by a substituting group. Here, as the alkyl group are given methyl group, ethyl group, n-propyl group, i-propyl group, n-butyl group, i-butyl group, s-butyl group, t-butyl group, pentyl group, isopentyl group, neopentyl group, hexyl group, heptyl group, octyl group, 2-ethylhexyl group, nonyl group, decyl group, undecyl group, dodecyl group, tridecyl group, isotridecyl group, myristyl group, palmityl group, stearyl group, icosyl group, docosyl group and the like, as the aryl group are given phenyl group, tolyl group, ethylphenyl group, xylyl group, cumenyl group, mesityl group, naphthyl group, biphenyl group and the like, as the alkenyl group are given vinyl group, allyl group, isopropenyl group and the like, as the aralkyl group are given benzyl group, phenethyl group and the like, and as the arylalkenyl group are given styryl group, cinnamyl group and the like. Also, as the substituting group of the above monovalent hydrocarbon group are given halogen atom, nitro group, alkoxy group and the like, and as the said halogen atom are given fluorine atom, chlorine atom, bromine atom and the like, and as the said alkoxy group are given methoxy group, ethoxy group, n-propoxy group, i-propoxy group, n-butoxy group, i-butoxy group, s-butoxy group, t-butoxy group and the like. Out of these, as the R⁷s of formula (IV), alkyl group and aryl group are preferable, and methyl group is especially preferable. Moreover, when the two R⁷s are hydrogen, the compound of formula (IV) is phosphonic acid, when one of the two R⁷s is hydrogen and the other is a monovalent group, the compound of formula (IV) is phosphonate monoester, and when he two R⁷s are both monovalent group, the compound of formula (IV) is phosphonate diester. Furthermore, the amount of phosphonic acid or its derivative represented by the above formula (IV) used is preferably in the range of 1 -10 equivalents (eq) in relation to the aldehyde represented by the above formula (III), and more preferably in the range of 1 - 1.2 equivalents (eq).

Also, in the above formula (V), R⁶ and R⁷s are the same as above, and when the two R⁷s are hydrogen, the compound of formula (V) is α-hydroxyphosphonic acid, when one of the two R⁷s is hydrogen and the other is a monovalent group, the compound of formula (V) is α-hydroxyphosphonate monoester, and when he two R⁷s are both monovalent group, the compound of formula (V) is α-hydroxyphosphonate diester. α-hydroxyphosphonic acid and its derivatives of the said formula (V) shows bioactivity, and can be used as enzyme inhibitor and the like.

The method of producing the α-hydroxyphosphonic acid and its derivatives of the present invention is generally performed in an organic solvent. As the said organic solvent are preferable aprotic organic solvents, and specifically, ethers such as tetrahydrofuran (THF), diethylether (Et₂O), diisopropylether (ⁱPr₂O) and the like can be given.

The method of producing the α-hydroxyphosphonic acid and its derivatives of the present invention is, though not limited specifically, performed preferably at -15°C to room temperature, and more preferably at -15°C to 0°C. The enantiomer excess of the product decreases whether the reaction temperature is too high or too low. Also, the reaction time is not limited specifically, and is selected arbitrarily according to the above reaction temperature.

### <Examples>

In the following, the present invention is explained in further detail using examples, but the present invention is not to be limited in any way by the following examples.

### (Ligand formation example 1)

A monoammonium salt (3.40 g, 22.56 mmol) represented by the above formula (XI) wherein X² is chlorine atom and the two R³s are bonded with each other to form a tetramethylene group and an aldehyde (5.034 g, 21.48 mmol) represented by the above formula (IX) wherein R¹ and R² are t-butyl group and R⁴ is hydrogen atom was dissolved in dehydrated methanol (100 ml) at room temperature and stirred for three hours. Next, NaBH₄ (2.03 g, 53.7 mmol) was added to the said solution at 0°C, the solution was stirred at room temperature for two hours, quenched by adding water, and extracted using diethylether. The extract was dried using anhydrous sodium sulfate, then concentrated. Under nitrogen atmosphere, the obtained residue and di-t-butyl-di-carbnate (5.45 ml, 23.60 mmol) was dissolved in ethanol (100 ml) at room temperature then stirred for about one hour to concentrate the said solution. When the obtained residue was separated by chromatograph separation (hexane: ethyl acetate = 9:1 - 17:3) using silica gel, a compound (6.24 g, 67 % yield) represented by the above formula (XIV) wherein R¹ and R² are t-butyl group, the two R³s are bonded with each other to form tetramethylene group, R⁴ is hydrogen atom, and A is t-butoxycarbonyl (Boc) group was obtained. The result of the IR measurement (KBr method) of the compound was 3317, 2955, 2862, 1701, 1510, 1481, 1454, 1390, 1364, 1317, 1236, 1171, 1107, 1016, and 872 cm⁻¹.

Next, the compound (5.626 g, 13.01 mmol) obtained in the way mentioned above and formaldehyde aqueous solution (1.23 ml, 16.27 mmol) was dissolved in methanol (80 ml) at room temperature. 10 % Pd/C (1.03 g) was added to the said solution, and under hydrogen atmosphere, the solution was stirred for about five hours then filtered on Celite pad, and the filtrate was concentrated under reduced pressure. After that, methanol (30 ml) and 3M hydrochloric acid (30 ml) was added to the obtained residue, the solution was stirred at room temperature for about 34 hours, then 3M sodium hydroxide aqueous solution (35 mol) was added and extracted using diethylether. The extract was dried on anhydrous sodium hydroxide then concentrated. The obtained extract and an aldehyde (3.042 g, 13.01 mmol) represented by the above formula (IX) wherein R¹ and R² are t-butyl group and R⁴ is hydrogen atom was dissolved at room temperature in methanol (about 100ml) and stirred for about five hours. The formed precipitate was obtained through filtration, cleansed using methanol, then vacuum dried for three hours at 50°C to obtain a compound (5.54 g, 76 % yield) represented by the following formula (XX): 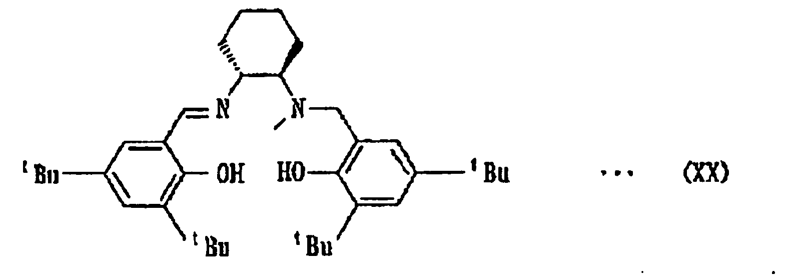 The result of the elemental analysis of the obtained compound was C: 78.94, H: 10.40, N: 4.92, and matched the calculated value of C₃₇H₅₈N₂O₂(C: 78.95, H: 10.39, N: 4.98).

### (Complex synthesis example 1)

A compound (453.4 mg, 0.806 mmol) represented by the above formula (XX) and hexane solution of Et₂AlCl (875.6 µl, 0.806 mmol) was dissolved at 0°C in toluene (10 ml), the said solution was stirred at 0°C for one hour then stirred at room temperature for 18 hours, and the solvent was distilled away under reduced pressure. After that, hexane was added to the obtained residue and the precipitation that formed was obtained by filtration through a glass filter and cleansed using hexane. By vacuum drying the precipitation obtained through filtration for three hours at 50°C, a compound (467.6 mg, 93 % yield) represented by the following formula (XXI): 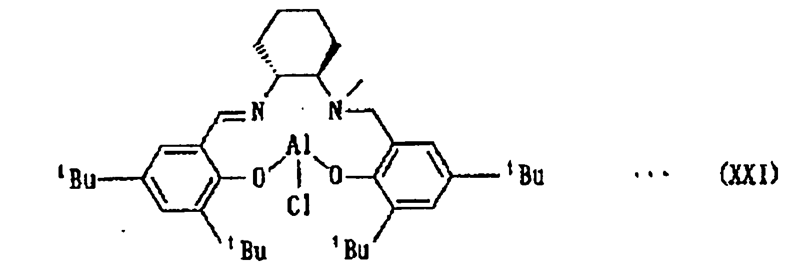 was obtained. The result of the elemental analysis of the obtained compound was C: 71.30, H: 9.03, N: 4.53, and matched the calculated value of C₃₇H₅₆N₂O₂ClAl (C: 71.35, H: 9.06, N: 4.49). Also, when the obtained complex was recrystallized from heptane/dichloromethane, a single crystal was obtained. The result of the X-ray structure analysis of the obtained crystal is shown in Fig. 1.

### (Example 1)

Under nitrogen atmosphere, the complex (12.5 mg, 0.02 mmol) represented by the above formula (XXI) and dimethyl phosphonate (10 µl, 0.21 mmol) was dissolved in THF (0.5 ml) and stirred at room temperature for 10 minutes. Next, benzaldehyde (0.20 mmol) was added at room temperature and the solution was further stirred for 24 hours. After that, 1M hydrochloric acid was added to stop the reaction, and extraction was performed three times using 1 ml of ethyl acetate. The obtained organic phase was let through Celite pad and sodium sulfate then the solvent was distilled away under reduced pressure. After that, the obtained residue was separated by chromatograph separation using silica gel and hexane/acetone (7/3 - 3/7) mixed solution, and the corresponding α-hydroxyphosphonate ester was obtained (92 % yield). Also, when the enantiomer excess of the obtained α-hydroxyphosphonate diester was analyzed by high performance liquid chromatography (HPLC) using chiral stationary phase column (Daicel Chiralpak AS-H) and hexane/isopropanol (4/1) mixed solution, the result was 73 %ee.

### (Examples 2 -9)

Except for changing the type of phosphonate diester and the solvent that is used, the reaction temperature, and reaction time as shown in Table 1, hydrophosphonylation of benzaldehyde was performed in the same way as Example 1 and each corresponding α-hydroxyphosphonate diester was produced. Also, the yield and the enantiomer excess were measured in the same way as in Example 1. The results are shown in Table 1.

**Table 1**

| | R⁷ of the phosphonate diester used *1 | Solvent | Reaction temperature (°C) | Reaction time (hours) | Yield (%) | Enantiomer excess (%ee) |
|---|---|---|---|---|---|---|
| Example 1 | methyl group | THF | room temp. | 24 | 92 | 73 |
| Example 2 | ethyl group | THF | room temp. | 24 | 96 | 70 |
| Example 3 | phenyl group | THF | room temp. | 24 | 87 | 17 |
| Example 4 | methyl group | Et₂O | room temp. | 24 | 97 | 68 |
| Example 5 | methyl group | ⁱPr₂O | room temp. | 24 | 94 | 79 |
| Example 6 | methyl group | THF | 0 | 24 | 91 | 87 |
| Example 7 | methyl group | ⁱPr₂O | 0 | 24 | 94 | 89 |
| Example 8 | methyl group | THF | -15 | 48 | 87 | 90 |
| Example 9 | methyl group | ⁱPr₂O | -15 | 48 | 80 | 81-90 *2 |

| | | | | | | |
|---|---|---|---|---|---|---|
| *1 The derivatives of phosphonic acid represented by formula (IV) *2 After testing in the same condition multiple times, there were variations in the enantiomer excess of the product | | | | | | |

As can be seen in Table 1, the production method of optically active α-hydroxyphosphonic acid and its derivatives of the present invention can be performed using various derivatives of phosphonic acid. Moreover, from the results of Examples 1-3, it can be seen that out of various derivatives of phosphonic acid, dialkyl phosphonate is preferable, and dimethyl phosphonate is expecially preferable. Also, it can be seen that the production method of optically active α-hydroxyphosphonic acid and its derivatives of the present invention can be performed in various organic solvents. Furthermore, it can be seen that the production method of optically active α-hydroxyphosphonic acid and its derivatives of the present invention can increase the enantioselectivity of the hydrophosphonylation when performed in lower temperatures, and that the range of 0 to -15°C is preferable.

### (Examples 10-17)

Except for using THF as the solvent, setting the reaction temperature at -15°C and the reaction time at 48 hours, and using the type of aldehydes shown in Table 2, hydrophosphonylation of each aldehyde was performed in the same way as Example 1 and each corresponding α-hydroxyphosphonate diester was produced. Also, the yield and the enantiomer excess were measured in the same way as in Example 1. Moreover, for Examples 10-15, Daicel Chiralpak AS-H and hexane/isopropanol (7/3) mixed solution was used for the measurement of enantiomer excess, and for Examples 16 and 17, Daicel Chiralpak AS-H and hexane/isopropanol (9/1) mixed solution was used.
The results are shown in Table 2.

**Table 2**

| | R⁶ of the aldehyde used | Yield (%) | Enantiomer excess (%ee) |
|---|---|---|---|
| Example 10 | p-O₂NC₆H₄- | 95 | 94 |
| Example 11 | p-ClC₆H₄- | 88 | 88 |
| Example 12 | p-CH₃OC₆H₄- | 87 | 81 |
| Example 13 | o-ClC₆H₄- | 96 | 91 |
| Example 14 | (E)-C₆H₅CH=CH- | 77 | 83 |
| Example 15 | C₆H₃CH₂CH₂- | 94 | 91 |
| Example 16 | (CH₃)₂CH- | 89 | 89 |
| Example 17 | CH₃CH₂- | 61 | 89 |

As can be seen in Table 2, in the production method of optically active α-hydroxyphosphonic acid and its derivatives of the present invention, the enantiomer excess of the product is high not only when using aromatic aldehydes, but also when using aliphatic aldehydes. Also, from the results of Examples 10-12, it can be seen that when using p-substituted benzaldehyde, the enantiomer excess of the product increases as the electron accepting ability of the substituting group at p-position increases.

### INDUSTRIAL APPLICABILITY

The production method of the present invention is very useful for asymmetrically hydrophosphonylating aldehydes by phosphonic acid or its derivatives to produce optically active α-hydroxyphosphonic acid or its derivatives. Also, the complexes of the present invention are very useful as the catalyst for the said production method. Moreover, the optically active α-hydroxyphosphonic acid and its derivatives obtained through the production method of the present invention have a unique bioactivity and is useful as medicinal chemicals such as enzyme inhibitors or their intermediates.

## Claims

1. A method of producing a optically active α-hydroxyphosphonic acid and its derivatives, **characterized in that** an optically active aluminium (salalen) complex represented by any one of the following formulae (I), (I') (II) and (II'): 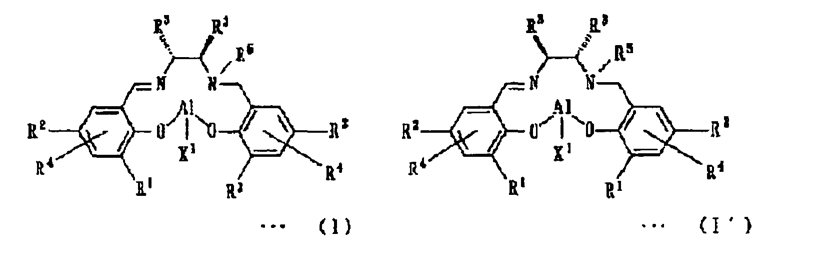 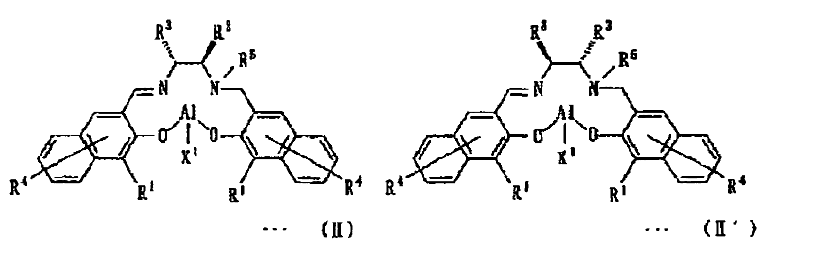 wherein each R¹ is independently alkyl or aryl group; each R² is independently an alkyl or aryl group; each R³ is independently an alky or aryl group, and two R³s may bond with each other to form a ring: each R⁴ is independently a hydrogen atom, halogen atom, alkyl group, alkoxy group, nitro group, or cyano group; R⁵ is an alkyl group; and X¹ is a halogen atom, alky group, alkoxy group, acetoxy group; and toluenesulfonyloxy group] is used as a catalyst and an aldehyde represented by the following formula (III): 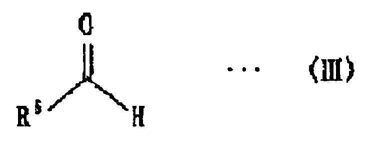 wherein R⁶ is an alkyl, aryl, alkenyl, aralkyl group or arylalkenyl group asymmetrically hydrophosphonylated with a phosphonic acid or its derivative represented by the following formula (IV): 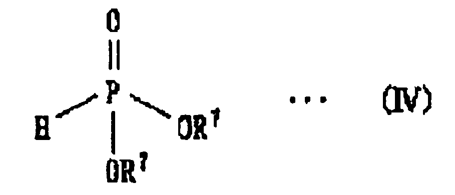 wherein each R⁷ is independently a hydrogen atom or alkyl, aryl, alkenyl, aralkyl or arylalkenyl group to produce optically active α-hydroxyphosphonic acid or its derivatives represented by the following formula (V): 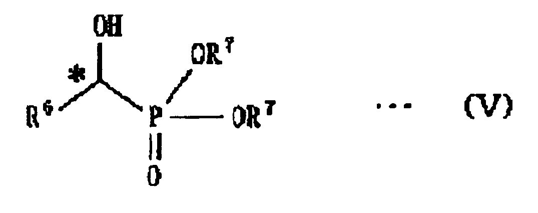 wherein R⁶ and R⁷s are as defined above.

2. A method of producing an optically active α-hydroxyphosphonic acid and its derivatives according to claim 1, wherein the two R³s in the said formulae are bonded with each other to form a tetramenthylene group.

3. A method of producing an optically active α-hydroxyphosphonic acid and its derivatives according to claim 1, wherein the said optically active aluminium (salalen) complex is represented by the above formula (I) or (I').

4. A method of producing an optically active α-hydroxyphosphonic acid and its derivatives according to claim 3, wherein R¹s and R²s in the said formulae are t-butyl group.

5. A method of producing an optically active α-hydroxyphosphonic acid and its derivatives according to claim 1, wherein R⁵ in the said formula is a methyl group.

6. A method of producing an optically active α-hydroxyphosphonic acid and its derivatives according to claim 1, wherein R¹s in the said formula (IV) are alkyl or aryl groups.

7. An optically active aluminium (salalen) complex represented by any one of the formulae (I), (I'), (II) and (II'), as defined in claim 1.

8. An optically active aluminium (salalen) complex according to claim 7, wherein the two R³s in the said formulae are bonded with each other to form a tetramethylene group.

9. An optically active aluminium (salalen) complex according to claim 7, which is represented by the said formula (I) or (I').

10. An optically active aluminium (salalen) complex according to claim 9, wherein R¹s and R²s in the said formulae are t-butyl groups.

11. An optically active aluminium (salalen) complex according to claim 7, wherein R⁵ in the said formula is a methyl group.

12. A method of producing an optically active aluminium (salalen) complex represented by any one of the said formulae (I), (I'), (II) and (II') as defined in claim 1, **characterized in that** a salalen ligand represented by any one of the following formulae (VI), (VI'), (VII) and (VII'). 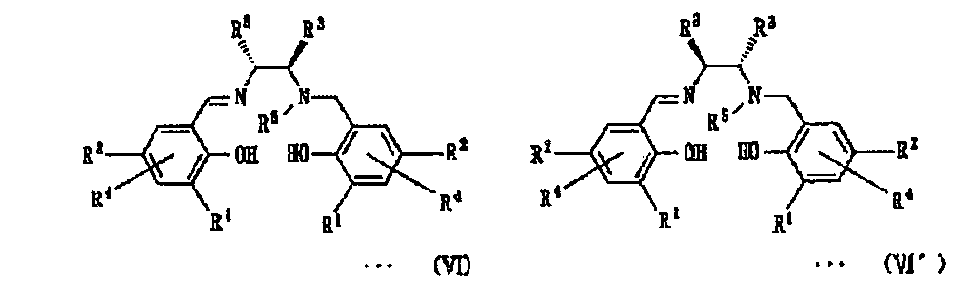 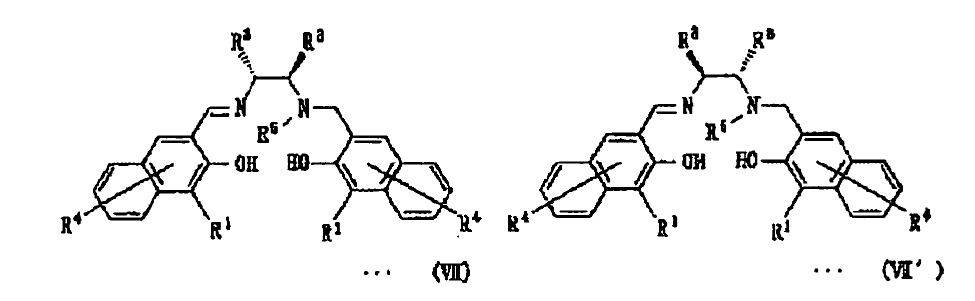 wherein R¹, R², R³, R⁴, and R⁵ are as defined in claim 1 is reacted with an aluminium compound represented by the following formula (VIII-a) or (VIII-b):
R⁸₂ A 1 X' (VIII-a)
R⁸₂ A 1 (VIII-b)
wherein each R⁸s is independently an alkyl group; and X¹ is as defined in claim 1.

13. A method of producing an optically active aluminium(salalen) complex according to claim 12, wherein the two R³s are bonded with each other to form a tetramethylene group:

14. A method of producing an optically active aluminium(salalen) complex according to claim 12, wherein the said salalen ligand is represented by formula (VI) or (VI'), and the said optically active aluminium(salalen) complex is represented by formula (I) or (I').

15. A method of producing an optically active aluminium(salalen) complex according to claim 14, wherein R¹s and R²s are t-butyl groups.

16. A method of producing an optically active aluminium(salalen) complex according to claim 12, wherein R⁵ is a methyl group.

17. A salalen ligand represented by any one of the said formulae (VI), (VI'), (VII) and (VII'), as defined in claim 12.

18. A salalen ligand according to claim 17, wherein the two R³s are bonded with each other to form a tetramethylene group.

19. A salalen ligand according to claim 17, which is represented by formula (VI) or (VI').

20. A salalen ligand according to claim 19, wherein R¹s and R²s are t-butyl groups.

21. A salalen ligand according to claim 17, wherein R⁵ is a methyl group.

22. A method of producing a salalen ligand, which comprises (i) a step of reductively aminating an aldehyde represented by the following formula (IX) or (X): 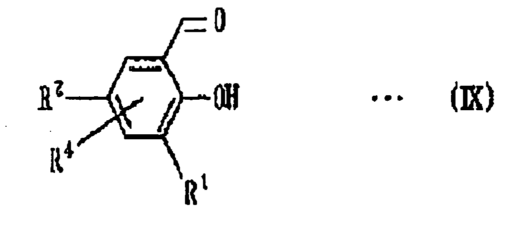 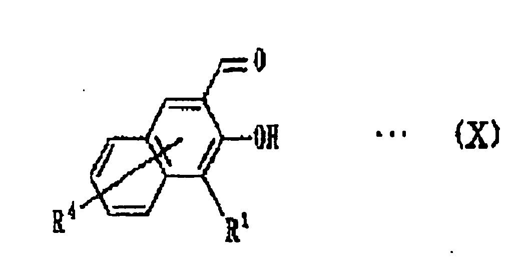 with monoammonium salt of a diamine represented by the following formula (XI) or (XI'): 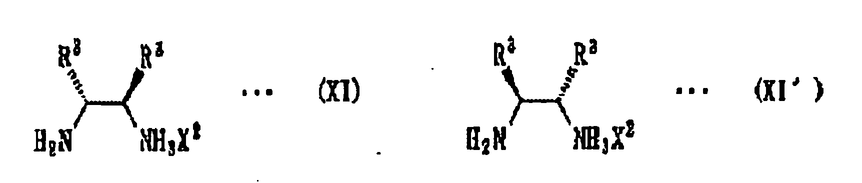 wherein X² is a halogen atom or an alkyl, alkoxy, acetoxy or teluenesulfonyloxy group and a reducing agent to form a compound represented by any one of the following formulae (XII), (XII'), (XIII) and (XIII'): 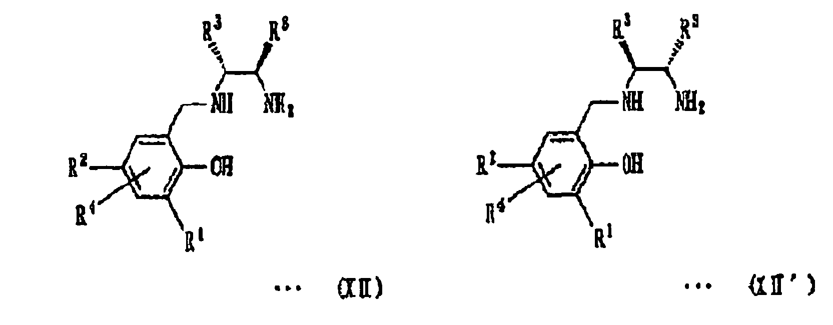 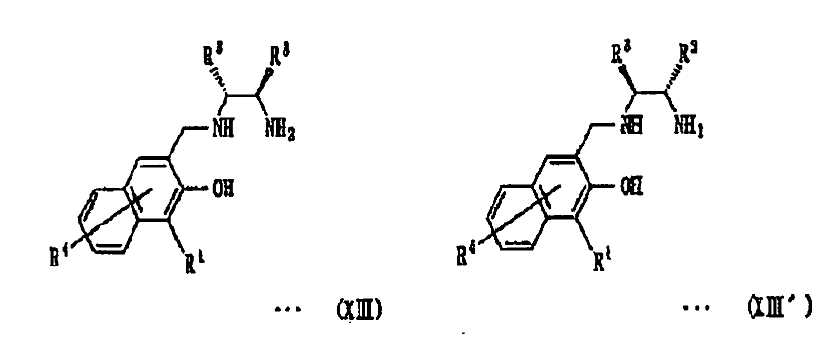 wherein R¹, R², R³, and R⁴ are as defined in claim 1;
(ii) a step of protecting an amino group of the compound represented by any one of the formulae (XII), (XII'), (XIII) and (XIII') with a protecting group A to form a compound represented by any one of the following formulae (XIV), (XIV'), (XV) and (XV'): 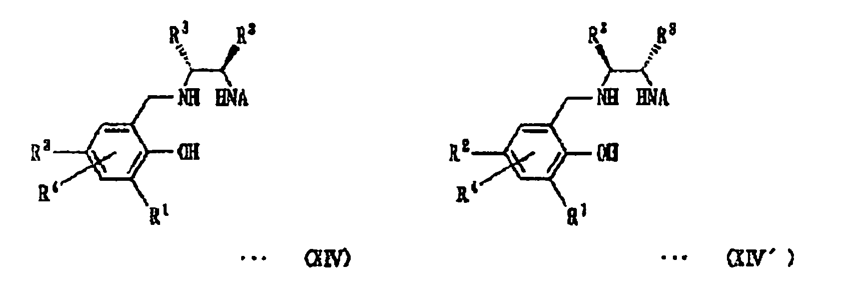 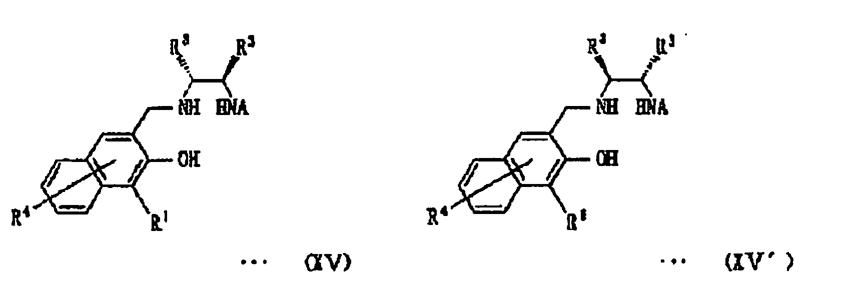 (iii) a step of N-alkylating the compound represented by any one of the formulae (XIV), (XIV'), (XV) and (XV') to form a compound represented by any one of the following formulae (XVI), (XVI'), (XVII) and (XVII'): 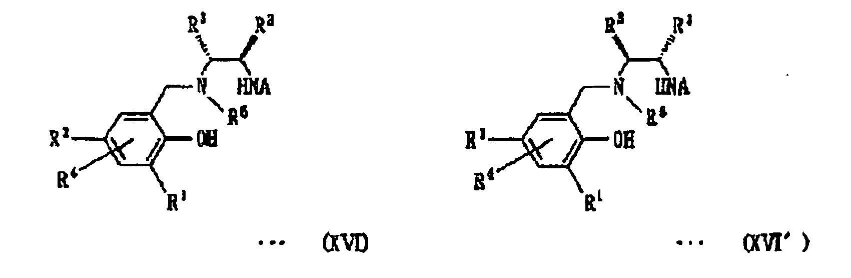 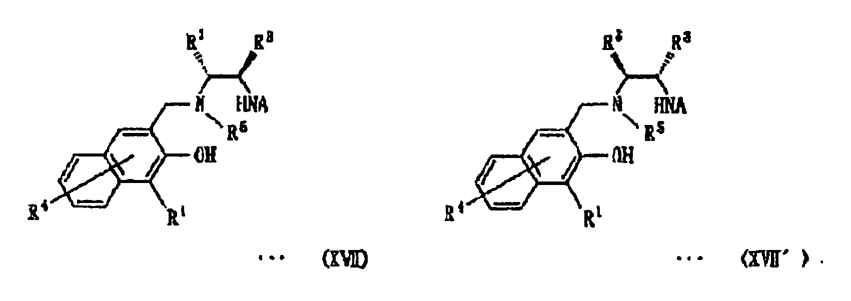 (iv) a step of deprotecting the protecting group in the compound represented by any one of the formulae (XVI), (XVI'), (XVII) and (XVII') to form a compound represented by any one of the following formulae (XVIII), (XVIII'), (XIX) and (XIX'): 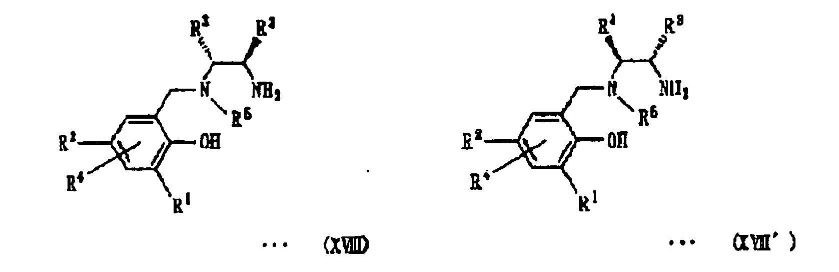 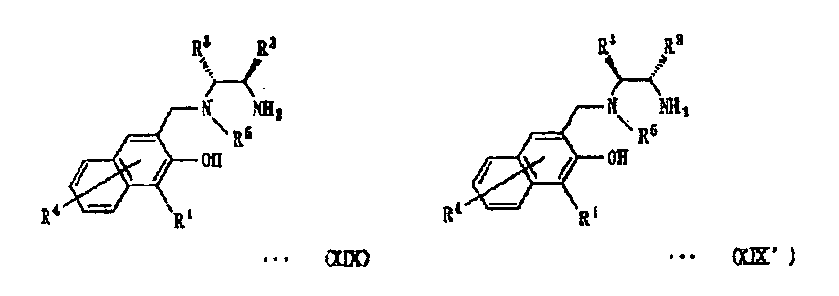 (v) a step of condensing the compound represented by any one of the formulae (XVIII), (XVIII'), (XIX) and (XIX') with the aldehyde represented by the formula (IX) or (X) to form a salalen ligand represented by any one of the formulae (VI), (VI'), (VII) and (VII'), as defined in claim 12.

23. A method of producing a salalen ligand according to claim 22, wherein the two R³s are bonded with each other to form a tetramethylene group.

24. A method of producing a salalen ligand according to claim 22, which is represented by formula (VI) or (VI').

25. A method of producing a salalen ligand according to claim 24, wherein R¹s and R²s are t-butyl groups.

26. A method of producing a salalen ligand according to claim 22, wherein the N-alkylation in the said step (iii) is N-methylation and the R⁵ is a methyl group.

## Patentansprüche

1. Verfahren zum Erzeugen einer optisch aktiven α-Hydroxyphosphonsäure und ihrer Derivate, **dadurch gekennzeichnet, dass** ein optisch aktiver Aluminium-(-Salalen-) -Komplex, der durch eine der folgenden Formeln (I), (I') , (II) und (II') dargestellt ist: 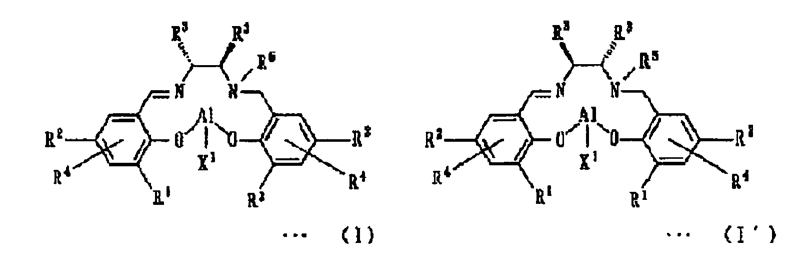 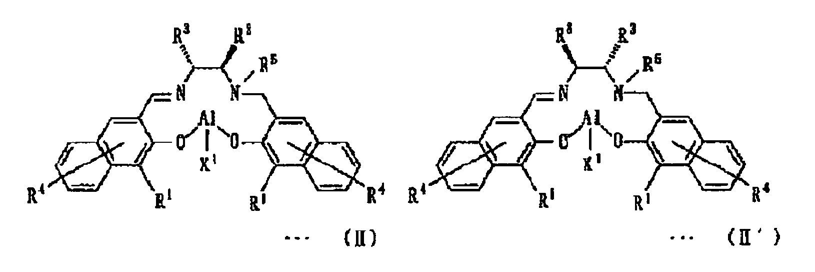 wobei jedes R¹ unabhängig eine Alkyl- oder Arylgruppe ist; jedes R² unabhängig eine Alkyl- oder Arylgruppe ist; jedes R³ unabhängig eine Alkyl- oder Arylgruppe ist und zwei R³s sich aneinander binden können, um einen Ring zu bilden; jedes R⁴ unabhängig ein Wasserstoffatom, Halogenatom, eine Alkylgruppe, Alkoxygruppe, Nitrogruppe oder Cyanogruppe ist; R⁵ eine Alkylgruppe ist und X¹ ein Halogenatom, eine Alkylgruppe, Alkoxygruppe, Acetoxygruppe; und
Toluensulfonyloxygruppe ist, als Katalysator verwendet wird und ein Aldehyd, das durch die folgende Formel (III) dargestellt ist: 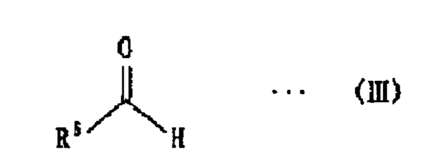 wobei R⁶ eine Alkyl-, Aryl-, Alkenyl-, Aralkylgruppe oder Arylalkenylgruppe ist, welche asymmetrisch mit einer Phosphorsäure oder deren Derivat hydrophosphonyliert wird, die bzw. das durch die folgende Formel (IV) dargestellt ist: 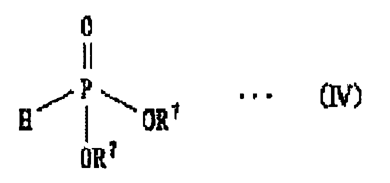 wobei jedes R⁷ unabhängig ein Wasserstoffatom oder eine Alkyl-, Aryl-, Alkenyl-, Aralkyl- oder Arylalkenylgruppe ist, um optisch aktive α-Hydroxyphosphonsäure oder deren Derivate zu erzeugen, die durch die folgende Formel (V) dargestellt ist bzw. sind: 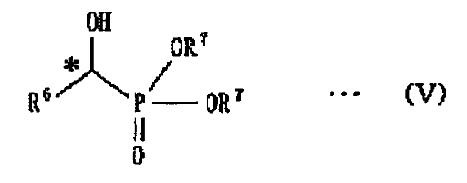 wobei R⁶ und R⁷s wie oben bestimmt sind.

2. Verfahren zum Erzeugen einer optisch aktiven α-Hydroxyphosphonsäure und ihrer Derivate gemäß Anspruch 1, wobei die zwei R³s in den Formeln aneinander gebunden sind, um eine Tetramethylengruppe zu bilden.

3. Verfahren zum Erzeugen einer optisch aktiven α-Hydroxyphosphonsäure und ihrer Derivate gemäß Anspruch 1, wobei der optisch aktive Aluminium- (-Salalen-) -Komplex durch die obige Formel (I) oder (I') dargestellt ist.

4. Verfahren zum Erzeugen einer optisch aktiven α-Hydroxyphosphonsäure und ihrer Derivate gemäß Anspruch 3, wobei R¹s und R²s in den Formeln eine t-Butylgruppe sind.

5. Verfahren zum Erzeugen einer optisch aktiven α-Hydroxyphosphonsäure und ihrer Derivate gemäß Anspruch 1, wobei R⁵ in der Formel eine Methylgruppe ist.

6. Verfahren zum Erzeugen einer optisch aktiven α-Hydroxyphosphonsäure und ihrer Derivate gemäß Anspruch 1, wobei R⁷s in der Formel (IV) Alkyl- oder Arylgruppen sind.

7. Optisch aktiver Aluminium- (-Salalen-) -Komplex, der durch eine der Formeln (I), (I'), (II) und (II'), wie in Anspruch 1 bestimmt, dargestellt ist.

8. Optisch aktiver Aluminium- (-Salalen-) -Komplex gemäß Anspruch 7, wobei die zwei R³s in den Formeln aneinander gebunden sind, um eine Tetramethylengruppe zu bilden.

9. Optisch aktiver Aluminium- (-Salalen-) -Komplex gemäß Anspruch 7, der durch die Formel (I) oder (I') dargestellt ist.

10. Optisch aktiver Aluminium- (-Salalen-) -Komplex gemäß Anspruch 9, wobei R¹s und R²s in den Formeln t-Butylgruppen sind.

11. Optisch aktiver Aluminium- (-Salalen-) -Komplex gemäß Anspruch 7, wobei R⁵ in der Formel eine Methylgruppe ist.

12. Verfahren zum Erzeugen eines optisch aktiven Aluminium-(-Salalen-) -Komplexes, der durch eine der Formeln (I), (I'), (II) und (II'), wie in Anspruch 1 bestimmt, dargestellt ist, **dadurch gekennzeichnet, dass** ein Salalen-Ligand, der durch eine der folgenden Formeln (VI), (VI'), (VII) und (VII') dargestellt ist, 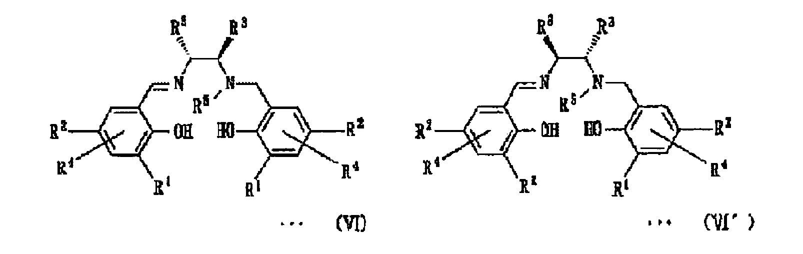 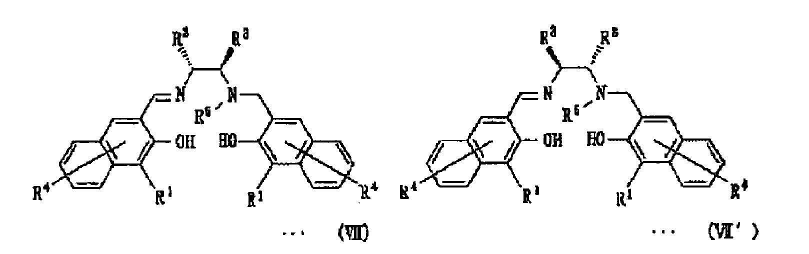 wobei R¹, R², R³, R⁴ und R⁵ wie in Anspruch 1 bestimmt sind, mit einer Aluminiumverbindung, dargestellt durch die folgende Formel (VIII-a) oder (VIII-b), in Reaktion gebracht wird:
R⁸₂ A 1 X' (VIII)-a)
R⁸₂ A 1 (VIII-b)
wobei jedes der R⁸s unabhängig eine Alkylgruppe ist und X¹ wie in Anspruch 1 bestimmt ist.

13. Verfahren zum Erzeugen eines optisch aktiven Aluminium- (-Salalen-) -Komplexes gemäß Anspruch 12, wobei die zwei R³s aneinander gebunden sind, um eine Tetramethylengruppe zu bilden.

14. Verfahren zum Erzeugen eines optisch aktiven Aluminium- (-Salalen-) -Komplexes gemäß Anspruch 12, wobei der Salalen-Ligand durch Formel (VI) oder (VI') dargestellt ist und der optisch aktive Aluminium- (-Salalen-) -Komplex durch Formel (I) oder (I') dargestellt ist.

15. Verfahren zum Erzeugen eines optisch aktiven Aluminium- (-Salalen-) -Komplexes gemäß Anspruch 14, wobei R¹s und R²s t-Butylgruppen sind.

16. Verfahren zum Erzeugen eines optisch aktiven Aluminium- (-Salalen-) -Komplexes gemäß Anspruch 12, wobei R⁵ eine Methylgruppe ist.

17. Salalen-Ligand, der durch eine der Formeln (VI), (VI'), (VII) und (VII') dargestellt ist, wie in Anspruch 12 bestimmt.

18. Salalen-Ligand gemäß Anspruch 17, wobei die zwei R³s aneinander gebunden sind, um eine Tetramethylengruppe zu bilden.

19. Salalen-Ligand gemäß Anspruch 17, der durch Formel (VI) oder (VI') dargestellt ist.

20. Salalen-Ligand gemäß Anspruch 19, wobei R¹s und R²s t-Butylgruppen sind.

21. Salalen-Ligand gemäß Anspruch 17, wobei R⁵ eine Methylgruppe ist.

22. Verfahren zum Erzeugen eines Salalen-Liganden, das (i) einen Schritt einer reduktiven Aminierung eines Aldehyds, das durch die folgende Formel (IX) oder (X) dargestellt ist: 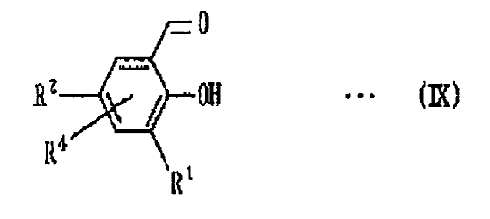 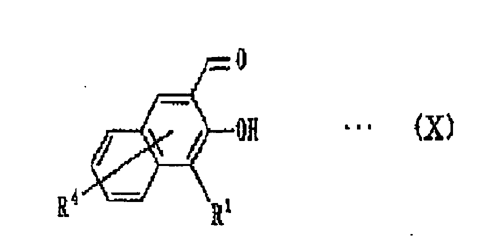 mit Monoammoniumsalz eines Diamins, das durch die folgende Formel (XI) oder (XI') dargestellt ist: 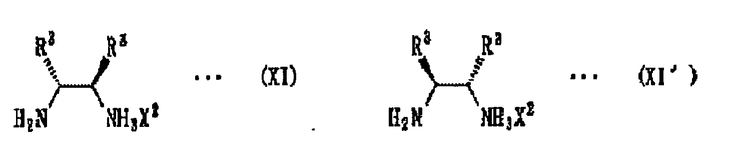 wobei X² ein Halogenatom oder eine Alkyl-, Alkoxy-, Acetoxy- oder Teluensulfonyloxygruppe ist, und einem Reduktionsmittel, um eine Verbindung zu bilden, die durch eine der folgenden Formeln (XII), (XII'), (XIII) und (XIII') dargestellt ist: 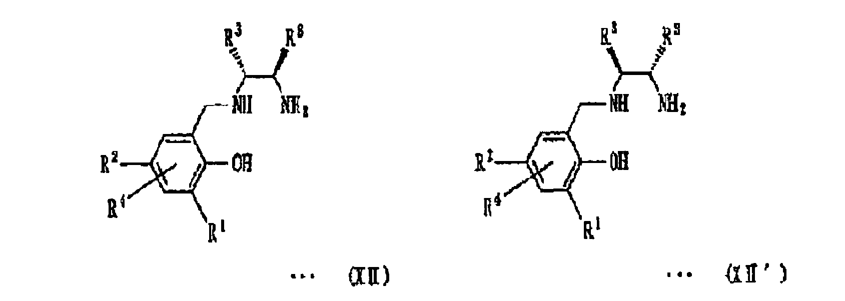 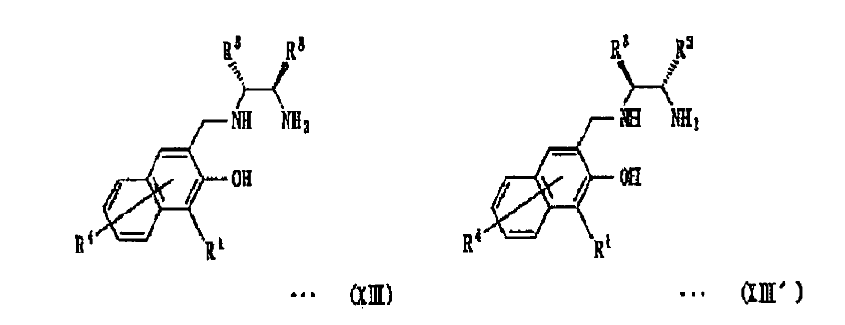 wobei R¹, R², R³ und R⁴ wie in Anspruch 1 bestimmt sind;
(ii) einen Schritt eines Schützens einer Aminogruppe der Verbindung, die durch eine der Formeln (XII), (XII'), (XIII) und (XIII') dargestellt ist, mit einer schützenden Gruppe A, um eine Verbindung zu bilden, die durch eine der folgenden Formeln (XIV), (XIV'), (XV) und (XV') dargestellt ist: 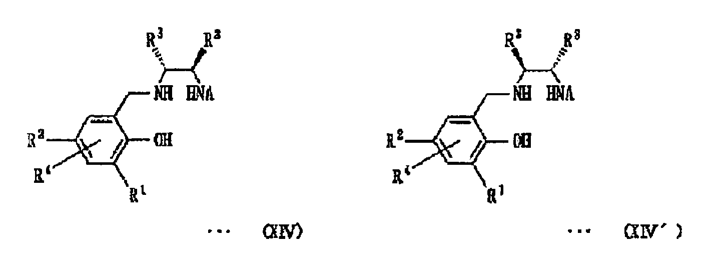 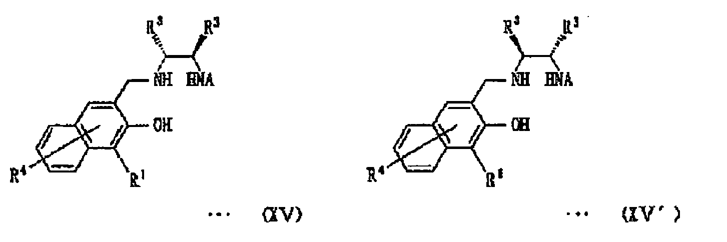 (iii) einen Schritt einer N-Alkylierung der Verbindung, die durch eine der Formeln (XIV), (XIV'), (XV) und (XV') dargestellt ist, um eine Verbindung zu bilden, die durch eine der folgenden Formeln (XVI), (XVI'), (XVII) und (XVII') dargestellt ist: 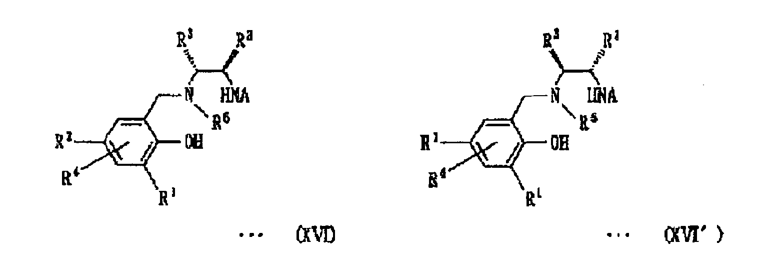 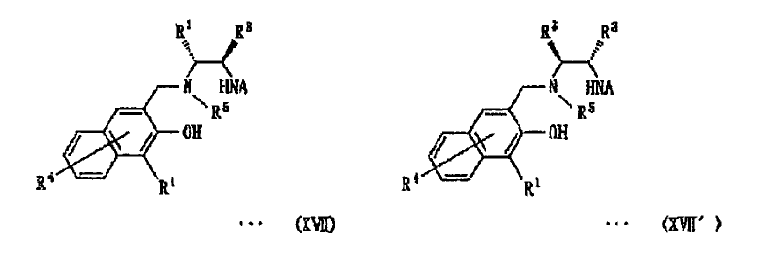 (iv) einen Schritt eines Aufhebens des Schutzes der schützenden Gruppe in der Verbindung, die durch eine der Formeln (XVI), (XVI'), (XVII) und (XVII') dargestellt ist, um eine Verbindung zu bilden, due durch eine der folgenden Formeln (XVIII), (XVIII'), (XIX) und (XIX') dargestellt ist: 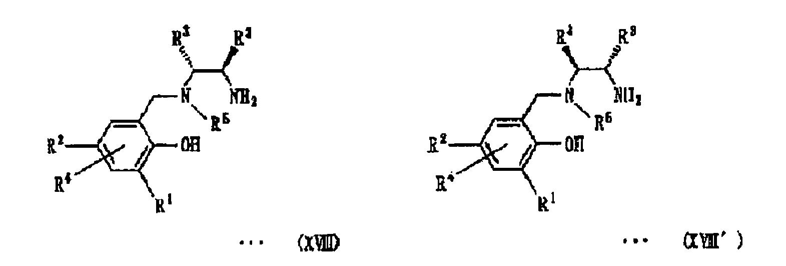 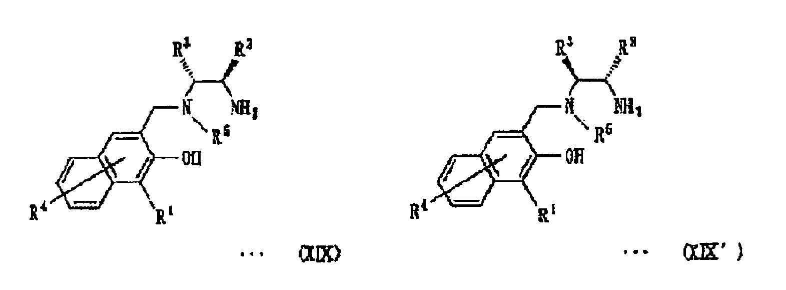 (v) einen Schritt eines Kondensierens der Verbindung, die durch eine der Formeln (XVIII), (XVIII'), (XIX) und (XIX') dargestellt ist, mit dem Aldehyd, das durch die Formel (IX) oder (X) dargestellt ist, um einen Salalen-Liganden zu bilden, der durch eine der Formeln (VI), (VI'), (VII) und (VII'), wie in Schritt 12 bestimmt, dargestellt ist, umfasst.

23. Verfahren zum Erzeugen eines Salalen-Liganden gemäß Anspruch 22, wobei die zwei R³s aneinander gebunden sind, um eine Tetramethylengruppe zu bilden.

24. Verfahren zum Erzeugen eines Salalen-Liganden gemäß Anspruch 22, der durch Formel (VI) oder (VI') dargestellt ist.

25. Verfahren zum Erzeugen eines Salalen-Liganden gemäß Anspruch 24, wobei R¹s und R²s t-Butylgruppen sind.

26. Verfahren zum Erzeugen eines Salalen-Liganden gemäß Anspruch 22, wobei die N-Alkylierung in dem Schritt (iii) N-Methylierung ist und das R⁵ eine Methylgruppe ist.

## Revendications

1. Procédé de production d'un acide α-hydroxyphosphonique optiquement actif et de ses dérivés, **caractérisé en ce qu'**un complexe d'aluminium (salalen) optiquement actif représenté par l'une quelconque des formules (I), (I'), (II) et (II') : 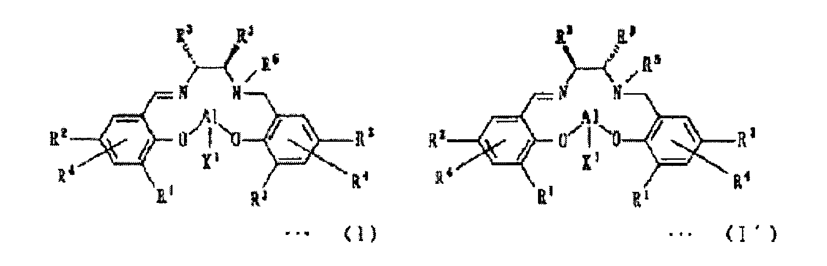 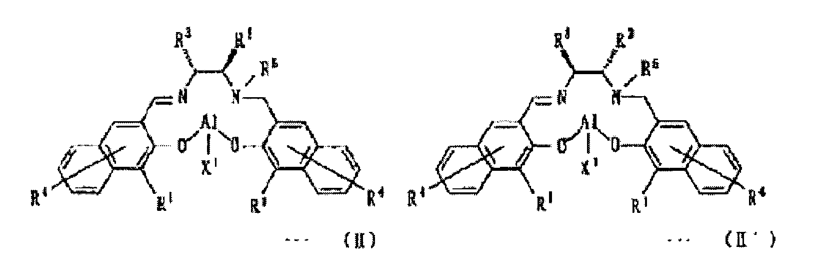 dans lesquelles chaque R¹ est indépendamment un groupe alkyle ou aryle ; chaque R² est indépendamment un groupe alkyle ou aryle ; chaque R³ est indépendamment un groupe alkyle ou aryle ; et deux R³ peuvent se lier l'un à l'autre pour former un cycle ; chaque R⁴ est indépendamment un atome d'hydrogène, un atome d'halogène, un groupe alkyle, un groupe alcoxy, un groupe nitro ou un groupe cyano ; R⁵ est un groupe alkyle ; et X¹ est un atome d'halogène, un groupe alkyle, un groupe alcoxy, un groupe acétoxy ; et un groupe toluènesulfonyloxy est utilisé comme catalyseur et un aldéhyde représenté par la formule (III) suivante : 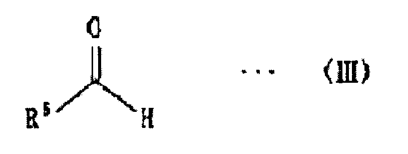 dans laquelle R⁶ est un groupe alkyle, aryle, alcényle, aralkyle ou arylalcényle asymétriquement hydrophosphonylé avec un acide phosphonique ou son dérivé représenté par la formule (IV) suivante : 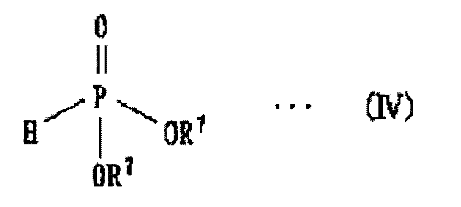 dans laquelle chaque R⁷ est indépendamment un atome d'hydrogène ou un groupe alkyle, aryle, alcényle, aralkyle ou arylalcényle pour produire un acide α-hydroxyphosphonique optiquement actif ou ses dérivés représentés par la formule (V) suivante : 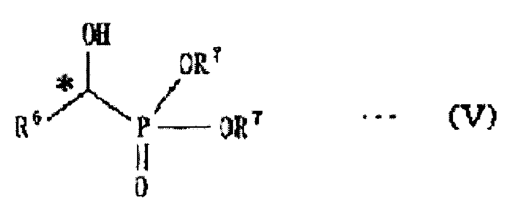 dans laquelle R⁶ et R⁷ sont tels que définis ci-dessus.

2. Procédé de production d'un acide α-hydroxyphosphonique optiquement actif et de ses dérivés selon la revendication 1, dans lequel les deux R³ dans lesdites formules sont liés l'un à l'autre pour former un groupe tétraméthylène.

3. Procédé de production d'un acide α-hydroxyphosphonique optiquement actif et de ses dérivés selon la revendication 1, dans lequel ledit complexe d'aluminium (salalen) optiquement actif est représenté par la formule (I) ou (I') ci-dessus.

4. Procédé de production d'un acide α-hydroxyphosphonique optiquement actif et de ses dérivés selon la revendication 3, dans lequel les R¹ et R² dans lesdites formules sont des groupes t-butyle.

5. Procédé de production d'un acide α-hydroxyphosphonique optiquement actif et de ses dérivés selon la revendication 1, dans lequel R⁵ dans ladite formule est un groupe méthyle.

6. Procédé de production d'un acide α-hydroxyphosphonique optiquement actif et de ses dérivés selon la revendication 1, dans lequel les R⁷ dans ladite formule (IV) sont des groupes alkyle ou aryle.

7. Complexe d'aluminium (salalen) optiquement actif représenté par l'une quelconque des formules (I), (I'), (II) et (II'), telles que définies dans la revendication 1.

8. Complexe d'aluminium (salalen) optiquement actif selon la revendication 7, dans lequel les deux R³ dans lesdites formules sont liés l'un à l'autre pour former un groupe tétraméthylène.

9. Complexe d'aluminium (salalen) optiquement actif selon la revendication 7, qui est représenté par ladite formule (I) ou (I').

10. Complexe d'aluminium (salalen) optiquement actif selon la revendication 9, dans lequel les R¹ et R² dans lesdites formules sont des groupes t-butyle.

11. Complexe d'aluminium (salalen) optiquement actif selon la revendication 7, dans lequel R⁵ dans ladite formule est un groupe méthyle.

12. Procédé de production d'un complexe d'aluminium (salalen) optiquement actif représenté par l'une quelconque desdites formules (I), (I'), (II) et (II'), telles que définies dans la revendication 1, **caractérisé en ce qu'**un ligand salalen représenté par l'une quelconque des formules (VI), (VI'), (VII) et (VII') suivantes : 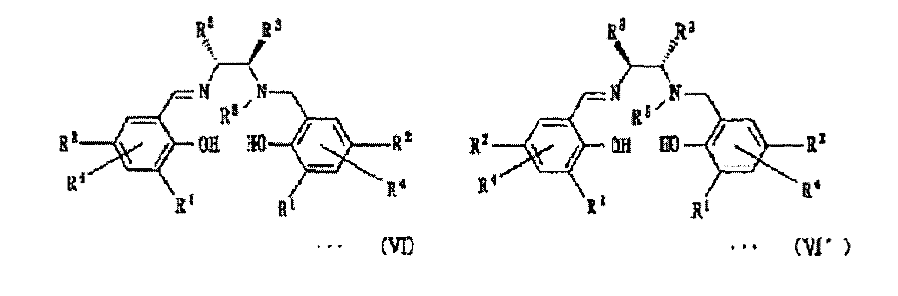 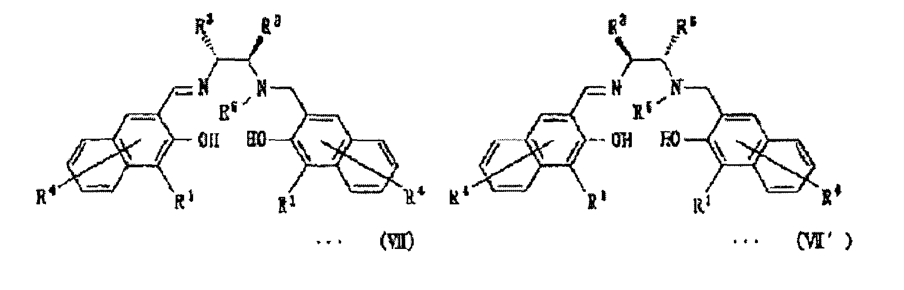 dans lesquelles R¹, R², R³, R⁴ et R⁵ sont tels que définis dans la revendication 1, réagit avec un composé aluminium représenté par la formule (VIII-a) ou (VIII-b) suivante :
R⁸₂A 1 X' (VIII-a)
R⁸₂A 1 (VIII-b)
dans laquelle chaque R⁸ est indépendamment un groupe alkyle ; et X¹ est tel que défini dans la revendication 1.

13. Procédé de production d'un complexe d'aluminium (salalen) optiquement actif selon la revendication 12, dans lequel les deux R³ sont liés l'un à l'autre pour former un groupe tétraméthylène.

14. Procédé de production d'un complexe d'aluminium (salalen) optiquement actif selon la revendication 12, dans lequel ledit ligand salalen est représenté par la formule (VI) ou (VI'), et ledit complexe d'aluminium (salalen) optiquement actif est représenté par la formule (I) ou (I').

15. Procédé de production d'un complexe d'aluminium (salalen) optiquement actif selon la revendication 14, dans lequel les R¹ et R² sont des groupes t-butyle.

16. Procédé de production d'un complexe d'aluminium (salalen) optiquement actif selon la revendication 12, dans lequel R⁵ est un groupe méthyle.

17. Ligand salalen représenté par l'une quelconque desdites formules (VI), (VI'), (VII) et (VII'), telles que définies dans la revendication 12.

18. Ligand salalen selon la revendication 17, dans lequel les deux R³ sont liés l'un à l'autre pour former un groupe tétraméthylène.

19. Ligand salalen selon la revendication 17, qui est représenté par la formule (VI) ou (VI').

20. Ligand salalen selon la revendication 19, dans lequel les R¹ et R² sont des groupes t-butyle.

21. Ligand salalen selon la revendication 17, dans lequel R⁵ est un groupe méthyle.

22. Procédé de production d'un ligand salalen, qui comprend (i) une étape d'amination réductrice d'un aldéhyde représenté par la formule (IX) ou (X) suivante : 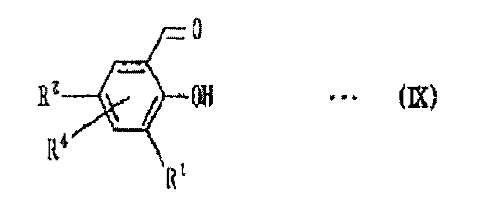 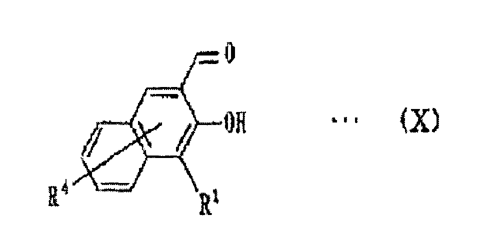 avec un sel de monoammonium d'une diamine représentée par la formule (XI) ou (XI') suivante : 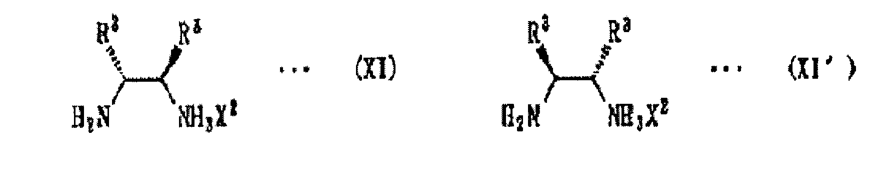 dans laquelle X² est un atome d'halogène ou un groupe alkyle, alcoxy, acétoxy ou toluènesulfonyloxy et un agent réducteur pour former un composé représenté par l'une quelconque des formules (XII), (XII'), (XIII) et (XIII') suivantes : 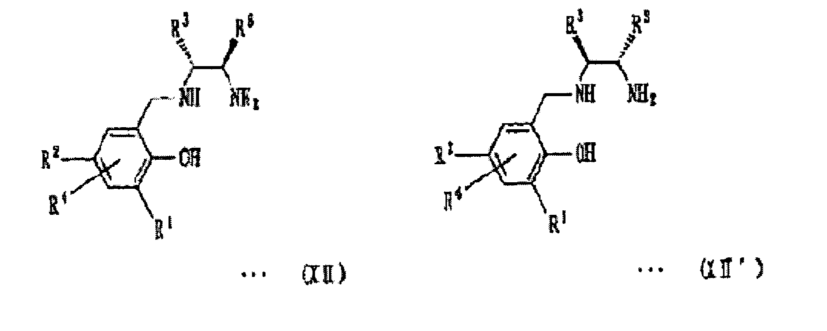 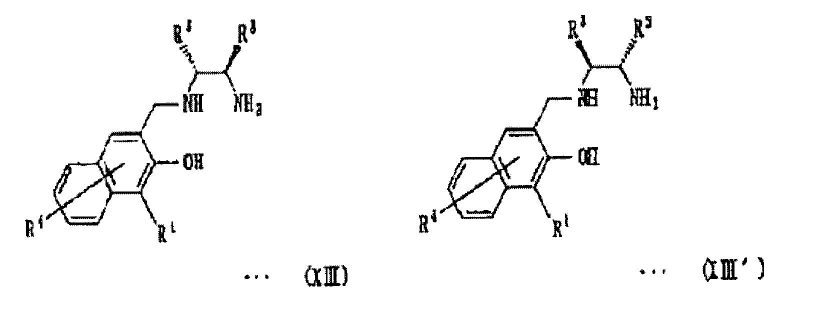 dans lesquelles R¹, R², R³ et R⁴ sont tels que définis dans la revendication 1 ;
(ii) une étape de protection d'un groupe amino du composé représenté par l'une quelconque des formules (XII), (XII'), (XIII) et (XIII') avec un groupe protecteur A pour former un composé représenté par l'une quelconque des formules (XIV), (XIV'), (XV) et (XV') suivantes : 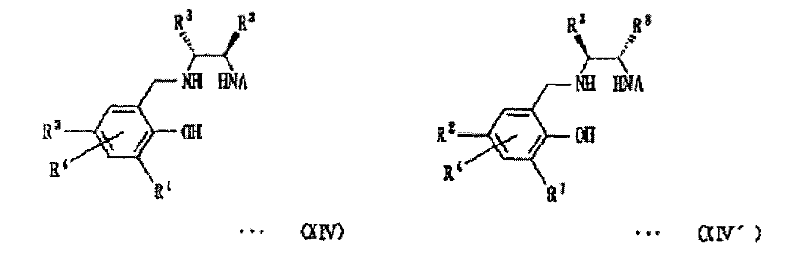 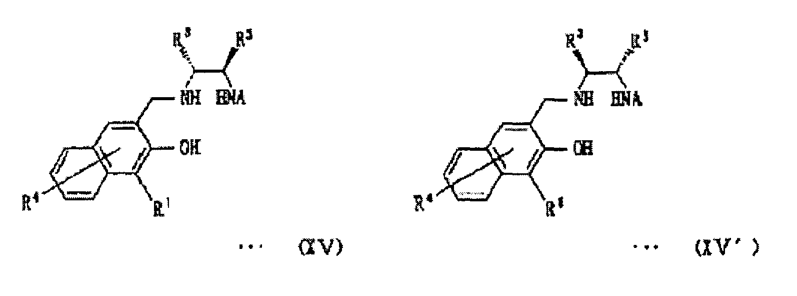 (iii) une étape de N-alkylation du composé représenté par l'une quelconque des formules (XIV), (XIV'), (XV) et (XV') pour former un composé représenté par l'une quelconque des formules (XVI), (XVI'), (XVII) et (XVII') suivantes : 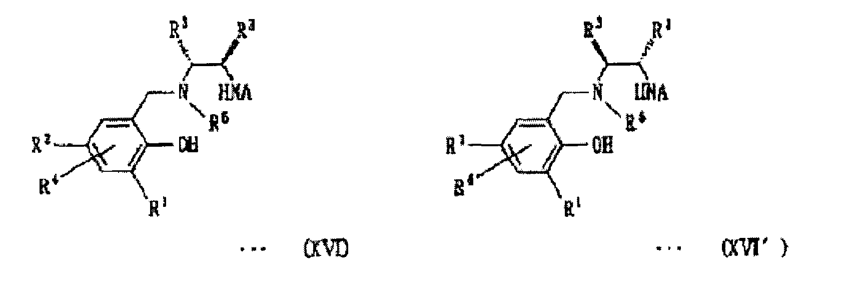 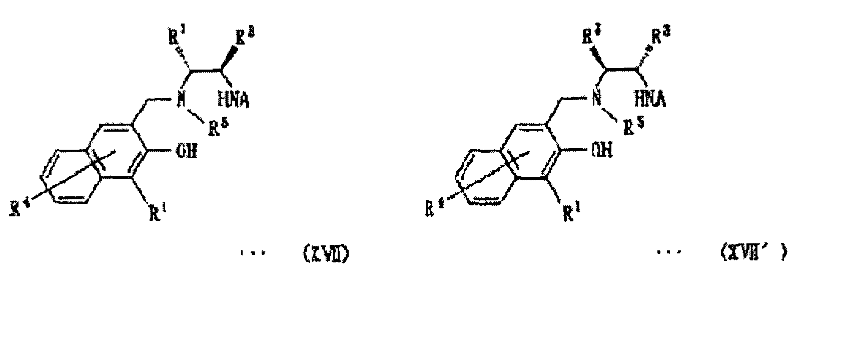 (iv) une étape de déprotection du groupe protecteur dans le composé représenté par l'une quelconque des formules (XVI), (XVI'), (XVII) et (XVII') pour former un composé représenté par l'une quelconque des formules (XVIII), (XVIII'), (XIX) et (XIX') suivantes : 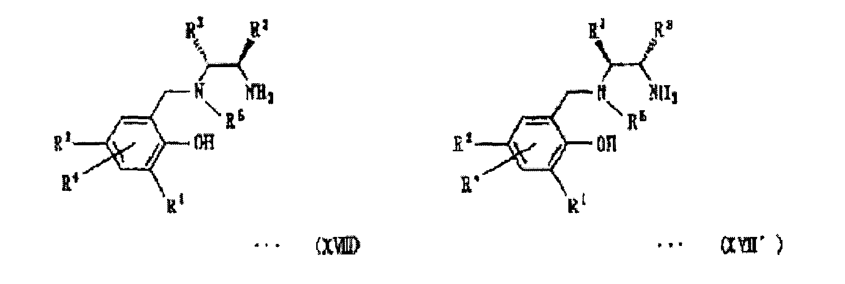 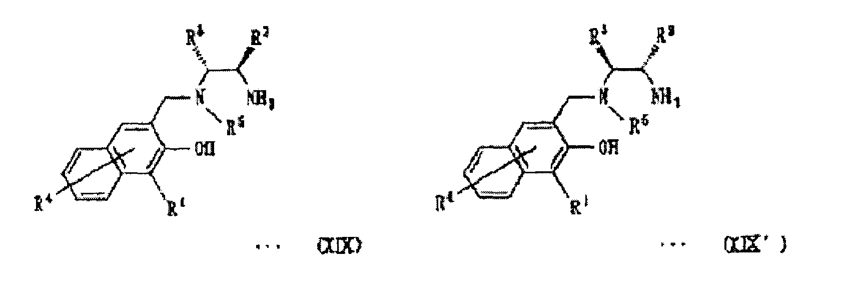 (v) une étape de condensation du composé représenté par l'une quelconque des formules (XVIII), (XVIII'), (XIX) et (XIX') avec l'aldéhyde représenté par la formule (IX) ou (X) pour former un ligand salalen représenté par l'une quelconque des formules (VI), (VI'), (VII) et (VII') telles que définies dans la revendication 12.

23. Procédé de production d'un ligand salalen selon la revendication 22, dans lequel les deux R³ sont liés l'un à l'autre pour former un groupe tétraméthylène.

24. Procédé de production d'un ligand salalen selon la revendication 22, qui est représenté par la formule (VI) ou (VI').

25. Procédé de production d'un ligand salalen selon la revendication 24, dans lequel les R¹ et R² sont des groupes t-butyle.

26. Procédé de production d'un ligand salalen selon la revendication 22, dans lequel la N-alkylation dans ladite étape (iii) est une N-méthylation et R⁵ est un groupe méthyle.
